(19) **Europäisches Patentamt / European Patent Office / Office européen des brevets**

(11) **EP 3 815 541 A1**

(12) # EUROPEAN PATENT APPLICATION
published in accordance with Art. 153(4) EPC

(43) Date of publication:
**05.05.2021 Bulletin 2021/18**

(21) Application number: **19803299.7**

(22) Date of filing: **17.05.2019**

(51) Int Cl.:
*A23L 29/00* (2016.01)   *A23K 20/163* (2016.01)
*A61K 8/73* (2006.01)   *A61K 47/38* (2006.01)
*A61Q 19/02* (2006.01)   *C08B 11/12* (2006.01)

(86) International application number:
**PCT/JP2019/019670**

(87) International publication number:
**WO 2019/221272 (21.11.2019 Gazette 2019/47)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.05.2018   JP 2018096479
28.03.2019   JP 2019063449**

(71) Applicant: **Nippon Paper Industries Co., Ltd.
Tokyo 114-0002 (JP)**

(72) Inventors:
• **INOUE, Kazuhiko**
  **Tokyo 114-0002 (JP)**

• **SUZUKI, Hiroyoshi**
  **Tokyo 114-0002 (JP)**
• **NISHIGAYA, Kasumi**
  **Tokyo 114-0002 (JP)**
• **TAKAYAMA, Masato**
  **Tokyo 114-0002 (JP)**
• **NAKADA, Sakiko**
  **Tokyo 114-0002 (JP)**
• **TAKAHASHI, Yuki**
  **Tokyo 114-0002 (JP)**
• **GOTO, Shisei**
  **Tokyo 114-0002 (JP)**

(74) Representative: **Cockerton, Bruce Roger
Carpmaels & Ransford LLP
One Southampton Row
London WC1B 5HA (GB)**

(54) **PULVERIZED PRODUCT OF CARBOXYMETHYLATED PULP AND ADDITIVE CONTAINING SAID PULVERIZED PRODUCT**

(57)   This pulverized product of carboxymethylated pulp has a carboxymethyl substation degree of 0.50 or less and a cellulose-I crystallinity index of 50% or more. This additive contains the pulverized product. Preferably, the carboxymethylated pulp is produced by performing a mercerization reaction in a solvent mainly containing water, and then performing a carboxymethylation reaction in a mixed solvent containing water and an organic solvent, and is fibrillated by wet pulverization.

EP 3 815 541 A1

**Description**

TECHNICAL FIELD

The present invention relates to a pulverized product of carboxymethylated pulp, and an additive containing the pulverized product. Specifically, the present invention relates to a pulverized product of carboxymethylated pulp having a specified degree of carboxymethyl substitution and a specified degree of crystallization of cellulose type I, and an additive containing the pulverized product.

BACKGROUND ART

Carboxymethylated pulp is obtained by linking carboxymethyl groups to some of hydroxyl groups in glucose residues in cellulose that constitutes a pulp to thereby form ether linkages. Carboxymethylated cellulose is used for various additives such as thickeners, caking agents, binders, water absorption materials, water retention materials, and emulsion stabilizers in, for example, cosmetic products, pharmaceutical products, food products, and various industrial products. Carboxymethylated cellulose is derived from native celluloses and thus is an environmentally friendly material which not only has mild biodegradability, but also can be burned and wasted, and thus applications thereof are expected to be expanded hereafter.

A commonly known method for producing carboxymethylated cellulose is a method including subjecting cellulose to an alkaline treatment (mercerization) and thereafter a treatment with an etherifying agent (also referred to as "carboxymethylating agent".) (carboxymethylation, also called "etherification".). A method including performing both mercerization and carboxymethylation in water as a solvent, and a method including performing both mercerization and carboxymethylation in a solvent containing mainly an organic solvent are known (PTLS 1 to 4). The former is called "water mediated method" and the latter is called "solvent mediated method".

It is known to defibrate carboxymethylated cellulose to a nano level to thereby provide a cellulose nanofiber. PTL 5 describes a carboxymethylated cellulose fiber which is obtained by defibrating carboxymethylated cellulose (having a degree of carboxymethyl substitution of 0.01 to 0.30) to have an average fiber diameter of 3 to 500 nm and an aspect ratio being 100 or more. The average fiber diameter is preferably 3 to 150 nm, further preferably 3 to 20 nm, further preferably 5 to 19 nm, further preferably 5 to 15 nm. Such a cellulose fiber defibrated to a nano level is called "cellulose nanofiber." In particular, a cellulose fiber having a fiber diameter around 4 nm corresponds to a single microfibril that is a base unit of cellulose bundles in cell walls of plants.

CITATION LIST

PATENT LITERATURE

PTL 1: Japanese Patent Laid-Open No. 2017-149901
PTL 2: Japanese Patent Laid-Open No. 2008-222859
PTL 3: Japanese Patent Laid-Open No. 2007-191558
PTL 4: Japanese Patent Laid-Open No. 2002-194001
PTL 5: International Publication No. WO 2014/088072

SUMMARY OF INVENTION

TECHNICAL PROBLEM

Carboxymethylated cellulose is used for additives in various fields of food/drink products, cosmetic products, aqueous paints, and the like because of having properties such as thickening properties, water absorption ability, and water retention ability. Such generally used carboxymethylated cellulose is usually a water-soluble polymer having a degree of carboxymethyl substitution (also referred to as "degree of etherification") of 0.55 of more. On the other hand, recently, studies have also been made for a carboxymethylated cellulose which has a degree of carboxymethyl substitution of 0.50 or less, which has crystallinity of cellulose remaining therein, and which is not completely dissolved in water and partially maintains a fibrous shape, and new applications utilizing the characteristics such as the shape and crystallinity thereof have been searched.

However, a carboxymethylated cellulose having a degree of carboxymethyl substitution of 0.50 or less and having a degree of crystallization of cellulose type I of 50% or more tends to be heterogeneous and has the problems of, for example, being unstable in dispersion and easily forming a clump in water. This is probably because carboxymethyl groups are locally introduced into cellulose to thereby cause a section soluble in water and a section insoluble in water to be locally generated in the carboxymethylated cellulose, resulting in the occurrence of variation in quality, and unstable dispersion depending on the state of introduction of carboxymethyl groups. Such a phenomenon remarkably occurs particularly when a degree of carboxymethyl substitution is low. It is considered that the reason is that small amounts of carboxymethyl groups are difficult to be uniformly introduced into cellulose (namely, such small amounts of carboxymethyl groups tend to be introduced locally). For example, if the degree of carboxymethyl substitution is in the range of 0.20 or more and 0.50 or less, it is difficult to achieve a degree of crystallization of cellulose type I of 50% or more. This is probably because carboxymethyl groups are locally introduced into cellulose to thereby cause dissolution in water from a section where substituents are collected, resulting in deterioration in crystallinity of the entire carboxymethylated cellulose.

The cellulose nanofiber described in PTL 5 is very fine and thus may be hard to use depending on the intended use. For example, such a cellulose nanofiber to be used as an additive for papermaking, when used in a small amount, easily slips thorough wires and can cause the problems of, for example, a low yield and a low paper strength. On the other hand, when used in a large amount, such a cellulose nanofiber can cause the problems of, for example, a too high water retention ability and thus poor water discharge of a pulp slurry in papermaking and a high cost. As a method for addressing such problems, it is considered to use a fiber lower in the degree of defibration than such a cellulose nanofiber; that is, a fiber having a lower degree of defibration; in other words, defibration is not conducted to a level of a single microfibril and defibration is suppressed to such an extent that a microfibril is fluffed from a cellulose fiber (fibrillation). However, such a fibrillated fiber may be remarkably thickened due to formation of a microfibril network, resulting in deterioration in handling properties and/or no favorable progression of fibrillation by itself (stickiness and/or clogging can occur in an apparatus).

An object of the present invention is to provide a material which mainly contains carboxymethylated pulp low in the degree of carboxymethyl substitution (0.50 or less) and high in the degree of crystallization of cellulose type I (50% or more), and which is homogeneous and hardly causes the above problems, and is suitable for use as an additive. Another object thereof is to provide a pulverized product of carboxymethylated pulp, which is relatively low in viscosity and favorable in handling properties.

SOLUTION TO PROBLEM

The present inventors have found that in a case where carboxymethylated pulp low in the degree of carboxymethyl substitution (0.50 or less) and high in the degree of crystallization of cellulose type I (50% or more) is produced by mercerization of pulp (alkaline treatment of cellulose) in a solvent containing mainly water and thereafter carboxymethylation (also referred to as "etherification".) in a mixed solvent of water and an organic solvent, homogeneous carboxymethylated pulp can be produced as compared with that obtained according to a conventional water mediated method (method including performing both mercerization and carboxymethylation in water as a solvent) or a solvent mediated method (method including performing both mercerization and carboxymethylation in a solvent containing mainly an organic solvent). It has also been found that a pulverized product obtained by pulverizing the carboxymethylated pulp produced according to the method has high water retention ability and favorable fluidity. It has also been found that an additive including the pulverized product of carboxymethylated pulp is homogeneous and excellent in dispersion stability, is excellent in imparting of water retention ability and shape retention ability, is relatively less sticky even in contact with water, and hardly forms a clump (aggregate) in water, and therefore can be suitably used in various fields.

The present invention provides the following, but is not limited thereto.

[1] A pulverized product of carboxymethylated pulp having a degree of carboxymethyl substitution of 0.50 or less and having a degree of crystallization of cellulose type I of 50% or more.

[2] The pulverized product of carboxymethylated pulp according to [1], wherein pulverization is wet pulverization.

[3] The pulverized product of carboxymethylated pulp according to [1] or [2], fibrillated by pulverization.

[4] The pulverized product of carboxymethylated pulp according to any one of [1] to [3], having an average fiber diameter of more than 500 nm.

[5] The pulverized product of carboxymethylated pulp according to any one of [1] to [4], having a degree of anionization of 0.10 meq/g or more and 2.00 meq/g or less.

[6] The pulverized product of carboxymethylated pulp according to any one of [1] to [5], having a degree of anionization of 1.00 meq/g or less.

[7] The pulverized product of carboxymethylated pulp according to any one of [1] to [6], having a viscosity (25°C, 60 rpm) of 2500 mPa•s or less when in the form of a water dispersion having a solid content of 1% by mass.

[8] The pulverized product of carboxymethylated pulp according to any one of [1] to [7], having a water retention capacity of 15 or more, as determined by preparing 40 mL of a slurry having a solid content of 0.3% by mass, with water, then centrifuging the slurry by a centrifuge at 30°C and 25000 G for 30 minutes to thereby separate an aqueous phase and a sediment, and performing calculation according to the following expression:

$$\text{Water retention capacity} = (B + C - 0.003 \times A)/(0.003 \times A - C)$$

wherein A represents a mass of the slurry subjected to centrifugation, B represents a mass of the sediment separated, and C represents a mass of a solid content in the aqueous phase separated.

[9] The pulverized product of carboxymethylated pulp according to any one of [1] to [8], having a structure obtained by linking carboxymethyl groups to some of hydroxyl groups in glucose residues in cellulose to thereby form ether linkages.

[10] An additive comprising the pulverized product of carboxymethylated pulp according to any one of [1] to [9].

[11] The additive according to [10], as an additive for a food product, an additive for a pharmaceutical product, an additive for a cosmetic product, an additive for a feed, an additive for papermaking, an additive for a paint, an agent imparting water retention ability, an agent imparting shape retention ability, a viscosity modifier, an emulsion stabilizer, or a dispersion stabilizer.

[12] A method for producing a pulverized product of carboxymethylated pulp having a degree of carboxymethyl substitution of 0.50 or less and having a degree of crystallization of cellulose type I of 50% or more, the method comprising

a step of obtaining a pulverized product of carboxymethylated pulp by a mechanical treatment of carboxymethylated pulp.

[13] The production method according to [12], wherein

the step of obtaining a pulverized product of carboxymethylated pulp by a mechanical treatment comprises fibrillating carboxymethylated pulp by a mechanical treatment, and

the pulverized product of carboxymethylated pulp comprises fibrillated carboxymethylated pulp.

[14] The production method according to [12] or [13], further comprising a step of subjecting pulp to a mercerization reaction in a solvent containing mainly water and thereafter a carboxymethylation reaction in a mixed solvent of water and an organic solvent to thereby produce carboxymethylated pulp, before the step of obtaining a pulverized product of carboxymethylated pulp by a mechanical treatment.

[15] The production method according to [14], wherein the solvent containing mainly water is a solvent comprising more than 50% by mass of water.

[16] The production method according to [14] or [15], wherein a proportion of the organic solvent in the mixed solvent is 50 to 99% by mass based on the total of water and the organic solvent.

## ADVANTAGEOUS EFFECTS OF INVENTION

The pulverized product of carboxymethylated pulp and the additive containing the same of the present invention are homogeneous and excellent in dispersion stability, are excellent in imparting of water retention ability and shape retention ability, are relatively less sticky even in contact with water, and hardly form a clump (aggregate) in water, and therefore can be suitably used for various additives such as an agent imparting water retention ability, an agent imparting shape retention ability, a viscosity modifier, an emulsion stabilizer, and a dispersion stabilizer in various fields of food products, pharmaceutical products, cosmetic products, feeds, papermaking, paints, and the like.

Due to a fiber diameter not too fine and proper fibrillation (fluff of microfibril) of a fiber, the pulverized product of carboxymethylated pulp of the present invention exhibits high water retention ability and proper thickening properties as compared with, for example, a carboxymethylated cellulose not defibrated or fibrillated, and, for example, also has an effect capable of imparting higher strength to paper even when used in a small amount in the form of an additive for papermaking, as compared with a cellulose nanofiber that is finely defibrated. It is possible to provide new effects and applications which have been hardly achieved by a conventional carboxymethylated cellulose that is not fibrillated or a conventional cellulose nanofiber that is finely defibrated.

## DESCRIPTION OF EMBODIMENTS

<Carboxymethylated pulp>

The present invention relates to a pulverized product of carboxymethylated pulp having a degree of carboxymethyl substitution of 0.50 or less and having a degree of crystallization of cellulose type I of 50% or more, and an additive containing the pulverized product. The carboxymethylated pulp has a structure obtained by linking carboxymethyl groups to some of hydroxyl groups in glucose residues in cellulose in such pulp to thereby form ether linkages. Carboxymethylated pulp may also be in the form of a salt, and the carboxymethylated pulp in the present invention encompasses a salt of the carboxymethylated pulp. Examples of the salt of the carboxymethylated pulp include a metal salt such as a sodium salt.

Examples of the pulp for use as a raw material of the carboxymethylated pulp include bleached pulp or unbleached pulp of, for example, wood, cotton, straw, bamboo, hemp, jute, or kenaf. The method for producing the bleached pulp or unbleached pulp is not particularly limited, and may be a mechanical method, a chemical method, or a combined intermediate method between these two methods. Examples of the bleached pulp or unbleached pulp classified according to the production method include mechanical pulp (thermomechanical pulp (TMP), groundwood pulp) and chemical pulp (sulfite pulp such as needle (softwood) unbleached sulfite pulp (NUSP) and needle bleached sulfite pulp (NBSP), and kraft pulp such as needle unbleached kraft pulp (NUKP), needle bleached kraft pulp (NBKP), leaf (hardwood) unbleached kraft pulp (LUKP), and leaf bleached kraft pulp (LBKP)). Dissolving pulp may also be used, besides papermaking pulp. Dissolving pulp is pulp chemically refined, and is mainly used in a dissolved state in chemicals and serves as a main raw material of an artificial fiber, cellophane, or the like.

The carboxymethylated pulp for use in the present invention has an average particle size of about 0.1 to 300 $\mu$m, preferably about 10 to 100 $\mu$m, without any limitation thereto. An average particle size of 0.1 $\mu$m or more facilitates production, and an average particle size of 300 $\mu$m or less facilitates uniform mixing with an object such as a food product or a pharmaceutical product. In the present invention, the carboxymethylated pulp is pulverized to thereby provide a pulverized product of the carboxymethylated pulp.

The carboxymethylated pulp for use in the pulverized product and the additive of the present invention preferably maintains at least a part of or all of its fibrous shape, even if dispersed in water. That is, the carboxymethylated pulp is preferably such that a fibrous substance can be observed when a water dispersion of the carboxymethylated pulp is observed with an electron microscope. In a case where such carboxymethylated pulp is subjected to X-ray diffraction measurement, a peak of a cellulose type I crystal can be observed.

Carboxyl groups (-COOH) derived from carboxymethyl groups in the carboxymethylated pulp may be modified appropriately, as long as the effects of the present invention are not impaired. Examples of such modification include hydrophobization by linking an amine-based compound or a phosphorus-based compound having an alkyl group, an aryl group, an aralkyl group, or the like to the carboxyl group.

The carboxymethylated pulp may carry a metal, as long as the effects of the present invention are not impaired. Carrying a metal means that an aqueous solution including a metal compound is brought into contact with the carboxymethylated pulp to allow the metal compound to link to carboxylate groups (-COO-) derived from carboxyl groups (-COOH) of the carboxymethylated pulp, by a coordination linkage or hydrogen linkage. Thus, a carboxymethylated pulp that contains a metal compound where metal ions derived from the metal compound are ionically linked can be obtained. Examples of such a metal compound can include a metal salt including ions of one or more metal elements selected from the group consisting of Ag, Au, Pt, Pd, Mn, Fe, Ti, Al, Zn, or Cu.

The carboxymethylated pulp for use in the pulverized product and the additive of the present invention has a degree of carboxymethyl substitution per anhydrous glucose unit of cellulose of 0.50 or less, preferably 0.40 or less. If a degree of such substitution of more than 0.50, dissolution in water easily occurs, and it is not possible to maintain its fibrous shape in water, resulting in reduction of the effect of, imparting shape retention ability, etc. A certain degree of carboxymethyl substitution is required for obtaining the effect of, imparting water retention ability and shape retention ability, etc. For example, if a degree of carboxymethyl substitution is less than 0.02, an advantage due to introduction of carboxymethyl groups sometimes may not be obtained depending on the intended use. Accordingly, the degree of carboxymethyl substitution is preferably 0.02 or more, further preferably 0.05 or more, further preferably 0.10 or more, further preferably 0.15 or more, further preferably 0.20 or more, further preferably 0.25 or more, further preferably 0.30 or more. In particular, when a degree of carboxymethyl substitution is in the range of 0.20 or more and 0.50 or less, it was difficult to obtain carboxymethylated pulp having a degree of crystallization of cellulose type I of 50% or more. However, the present inventors have found that carboxymethylated pulp having a degree of carboxymethyl substitution of 0.20 or more and 0.50 or less and a degree of crystallization of cellulose type I of 50% or more can be obtained by, for example, a production method described below. Such carboxymethylated pulp is homogeneous, and hardly forms a clump in water. The degree of carboxymethyl substitution can be adjusted by controlling, for example, the amount of a carboxymethylating agent to be added in a reaction, the amount of a mercerizing agent, and the compositional ratio of water and an organic solvent.

The anhydrous glucose unit in the present invention means each anhydrous glucose (glucose residue) included in cellulose. The degree of carboxymethyl substitution (also referred to as "degree of etherification".) means the proportion of hydroxyl groups replaced with carboxymethyl ether groups among hydroxyl groups of glucose residues included in cellulose (the number of carboxymethyl ether groups per glucose residue). The degree of carboxymethyl substitution may be here abbreviated as DS.

The method for measuring the degree of carboxymethyl substitution is as follows:

About 2.0 g of a sample is precisely weighed and placed in a 300-mL stoppered conical flask. 100 mL of nitric acid/methanol (a liquid obtained by adding 100 mL of conc. nitric acid (special grade) to 1000 mL of methanol) is added thereto, and the resultant is shaken for 3 hours, thereby converting a salt of carboxymethylated cellulose (CMC) to H-CMC (hydrogen-type carboxymethylated cellulose). The absolute dry H-CMC is precisely weighed in an amount of 1.5 to 2.0 g and placed in a 300-mL stoppered conical flask. The H-CMC is wetted with 15 mL of 80% methanol, 100 mL of 0.1 N-NaOH is added thereto, and the resultant is shaken at room temperature for 3 hours. Phenolphthalein is used as an indicator to reversely titrate excess NaOH by 0.1 N-$H_2SO_4$, and the degree of carboxymethyl substitution (DS value) is calculated according to the following expressions.

$$A = [(100 \times F' - 0.1 \ N\text{-}H_2SO_4 \ (mL) \times F) \times 0.1]/(\text{Absolute dry mass (g) of H-CMC})$$

$$\text{Degree of carboxymethyl substitution} = 0.162 \times A/(1 - 0.058 \times A)$$

F': factor of 0.1 N-$H_2SO_4$
F: factor of 0.1 N-NaOH.

The degree of crystallization of cellulose in the carboxymethylated pulp for use in the pulverized product and the additive of the present invention is 50% or more, more preferably 60% or more, with respect to crystal I type. In a case where crystallinity is adjusted in the above range, the effect of imparting shape retention ability, etc. by the carboxymethylated pulp can be highly obtained. The crystallinity of cellulose can be controlled by the concentration of a mercerizing agent and the temperature in a treatment, as well as the degree of carboxymethylation. An alkali at a high concentration is used in mercerization and carboxymethylation to thereby allow a type I crystal of cellulose to be easily converted into a type II crystal; however, for example, the amount of the alkali (mercerizing agent) to be used can be adjusted to thereby adjust the degree of modification, thereby allowing desired crystallinity to be maintained. The upper limit of the degree of crystallization of cellulose type I is not particularly limited. The upper limit is considered to be actually about 90%.
The method for measuring the degree of crystallization of cellulose type I is as follows:
A sample is put on a glass cell and subjected to measurement with an X-ray diffraction diffractometer (LabX XRD-6000, manufactured by Shimadzu Corporation). The degree of crystallization is calculated according to a procedure of Segal, et al., and from the diffraction intensity of the 002 plane at $2\theta = 22.6°$ and the diffraction intensity of an amorphous portion at $2\theta = 18.5°$ with the diffraction intensity at $2\theta = 10°$ to $30°$ as the baseline in an X-ray diffraction diagram, according to the following expressions.

$$Xc = (I002c - Ia)/I002c \times 100$$

Xc = degree of crystallization (%) of cellulose type I
1002c: diffraction intensity of 002 plane at $2\theta = 22.6°$
Ia: diffraction intensity of amorphous portion at $2\theta = 18.5°$.

The carboxymethylated pulp preferably less forms a clump (aggregate) (namely, low formation of a filtration residue), when in the form of a dispersion with water as a dispersing medium (that is, water dispersion). Specifically, in a case where the carboxymethylated pulp is added to 500 g of water and the resultant is stirred at 400 rpm for 5 seconds and thereafter naturally filtered with a 20-mesh filter, the dry mass of a filtration residue on the filter is preferably 0 to 30% by mass based on the dry mass of the carboxymethylated pulp added to water (the proportion of the dry mass of the filtration residue after the natural filtration based on the dry mass of the carboxymethylated cellulose added to water, calculated according to the above method, is herein called "the proportion of the filtration residue".). A specific method for measuring the proportion of the filtration residue is as follows:

(1) Measurement of amount of filtration residue

500 g of water is collected in a 1-L beaker. Five grams of the carboxymethylated pulp is collected and the mass thereof is recorded (mass of carboxymethylated pulp). An impeller is installed to a stirrer (IKA® EUROSTAR P CV S1 (manufactured by IKA Corporation)), and water is stirred at 400 rpm. The carboxymethylated pulp whose mass is recorded is added to the stirred water at once, and the resultant is stirred for 5 seconds after the addition. After completion of the stirring, the stirrer is turned off. After completion of the stirring, natural filtration is rapidly performed with a 20-mesh filter whose mass is measured in advance. After the natural filtration, the filter and a residue thereon are dried together on a tray at 100°C for 2 hours. The mass of the filter and the residue thereon is measured, and the mass of the filter is subtracted therefrom, thereby calculating the absolute dry mass (g) of the residue (absolute dry mass of residue).

(2) Calculation of amount of moisture in carboxymethylated pulp

A weighing bottle is heated at 100°C for 2 hours and cooled in a desiccator with silica gel therein, and the absolute dry mass of the weighing bottle is precisely weighed (absolute dry mass of weighing bottle). About 1.5 g of the carboxymethylated pulp is metered and taken in the weighing bottle, and precisely weighed (mass of CMC before drying). The lid of the weighing bottle is opened, and heating and drying are made at 105°C for 2 hours. The lid of the weighing bottle is closed, and cooling is made in the desiccator with silica gel therein, for 15 minutes. The mass of the weighing bottle after drying (including the carboxymethylated pulp after drying) is precisely weighed (mass of weighing bottle including CMC after drying). The amount of moisture in the carboxymethylated pulp is calculated according to the following expression:

Moisture (%) in carboxymethylated pulp = [{Mass (g) of CMC before drying - (Mass (g) of weighing bottle including CMC after drying - Absolute dry mass (g) of weighing bottle)}/Mass (g) of CMC before drying] $\times$ 100.

(3) Calculation of proportion of filtration residue

The proportion of the filtration residue of the carboxymethylated pulp is calculated using the mass (g) of the carboxymethylated cellulose and the absolute dry mass (g) of the residue, measured in (1), and the moisture (%) in the carboxymethylated pulp, calculated in (2), according to the following expression:

Proportion (%) of filtration residue of carboxymethylated pulp = [Absolute dry mass (g) of residue/{Mass (g) of carboxymethylated pulp $\times$ (100 - Moisture (%) of carboxymethylated pulp)/100}] $\times$ 100.

The proportion of the filtration residue of the carboxymethylated pulp, calculated according to the above expression, is preferably 0 to 30%, further preferably 0 to 20%, further preferably 0 to 10%. A carboxymethylated pulp low in proportion of the filtration residue is easily dispersed and excellent in handling properties. Such carboxymethylated pulp low in proportion of the filtration residue can be produced according to, for example, a method described below.

The carboxymethylated pulp preferably has a Schopper-Riegler freeness of 60.0°SR or more. The method for measuring the Schopper-Riegler freeness of the carboxymethylated pulp is according to JIS P 82121-1: 2012, and is specifically as follows:

The carboxymethylated pulp is dispersed in water to prepare a water dispersion having a solid content of 10 g/L, and the water dispersion is stirred with a magnetic stirrer at 1000 rpm all night and all day. The resulting slurry is diluted to 1 g/L. A 60-mesh screen (wire diameter: 0.17 mm) is installed to DFR-04 manufactured by Mutec Co., Ltd., the amount of a liquid passing through the mesh, in 1000 ml of a testing liquid, is measured for 60 seconds, and the Schopper-Riegler freeness is calculated by the method according to JIS P 8121-1: 2012.

The Schopper-Riegler freeness is for measurement of the degree of water discharge of a fiber suspension, and the lower limit value is 0°SR, the upper limit value is 100°SR. It is indicated that, as the Schopper-Riegler freeness is closer to 100°SR, water discharge (amount of draining) is less made, namely, the water retention ability of a fiber is higher.

The carboxymethylated pulp preferably has a Schopper-Riegler freeness of 60.0°SR or more, further preferably 65.0°SR or more. The upper limit is not particularly limited, and is 100.0°SR or less, preferably, 90.0°SR or less. Carboxymethylated pulp having a Schopper-Riegler freeness of 60.0°SR or more is high in water retention ability, and can be said to be suitable for use as, for example, a water retention agent for various compositions such as food products, cosmetic products, and pharmaceutical products, without any limitation thereto. The carboxymethylated pulp having such a Schopper-Riegler freeness can be produced by, for example, a method described below.

The carboxymethylated pulp preferably has a Canadian standard freeness of 150 ml or less, more preferably 120 ml or less, further preferably 110 ml or less. The carboxymethylated pulp having such a Canadian standard freeness can be produced by, for example, a method described below. The Canadian standard freeness is for measurement of the degree of water discharge of a fiber suspension, and it is indicated that, as the value is smaller, water discharge (amount of draining) is less made, namely, the water retention ability of a fiber is higher. The method for measuring the Canadian standard freeness is as follows:

A sample is prepared according to the same method as in the above Schopper-Riegler freeness, a 60-mesh screen (wire diameter: 0.17 mm) is installed to DFR-04 manufactured by Mutec Co., Ltd., the amount of a liquid passing through the mesh, in 1000 ml of a testing liquid, is measured for 60 seconds, and the Canadian standard freeness is calculated by the method according to JIS P 8121-2: 2012.

The carboxymethylated pulp preferably has an amount of drainage of 400 ml or less, more preferably 380 ml or less, further preferably 370 ml or less. The carboxymethylated pulp having such an amount of drainage can be produced by, for example, a method described below. The amount of drainage is for measurement of the degree of water discharge of a fiber suspension It is indicated that, as the value is smaller, water discharge (amount of draining) is less made, namely, the water retention ability of a fiber is higher. The method for measuring the amount of drainage is as follows:

A sample is adjusted according to the same method as in the above Schopper-Riegler freeness, a 60-mesh screen (wire diameter: 0.17 mm) is installed to DFR-04 manufactured by Mutec Co., Ltd., the amount of a liquid passing through the mesh, in 1000 ml of a testing liquid, is measured for 60 seconds, and the amount of drainage is calculated.

The carboxymethylated pulp preferably exhibits a low viscosity when in the form of a dispersion using water as a dispersing medium (water dispersion). The method for measuring the viscosity of the carboxymethylated pulp is as follows:

The carboxymethylated pulp is metered and taken in a glass beaker having a volume of 1000 ml, and dispersed in 900 ml of distilled water, thereby preparing a water dispersion so that the solid content is 1% (w/v). The water dispersion is stirred at 25°C with a stirring machine at 600 rpm for 3 hours. Thereafter, the viscosity at a rotational speed of 30 rpm with a No.1 rotor after 3 minutes is measured with a B-type viscometer (manufactured by Toki Sangyo Co., Ltd.) according to the method of JIS Z 8803.

The carboxymethylated pulp preferably has a viscosity of 10.0 mPa•s or less, more preferably 8.0 mPa•s or less, further preferably 7.0 mPa•s or less. Carboxymethylated pulp having such a low viscosity is considered to have carboxymethyl groups not locally, but uniformly introduced into the entire cellulose, and is considered to be able to further stably obtain a unique effect for the carboxymethylated pulp, for example, the effect of imparting shape retention ability and water absorption ability, etc. The carboxymethylated pulp having such a viscosity can be produced by, for example, a method described below. The lower limit value of the viscosity is not particularly limited, and the lower limit is considered to be actually about 1.0 mPa•s.

The carboxymethylated pulp preferably has a degree of anionization (also referred to as "density of anionic charge".) of 1.00 meq/g or less, preferably 0.00 meq/g or more and 1.00 meq/g or less. The method for measuring the degree of anionization of the carboxymethylated pulp is as follows:

The carboxymethylated pulp is dispersed in water to prepare a water dispersion having a solid content of 10 g/L, and the water dispersion is stirred with a magnetic stirrer at 1000 rpm all night and all day. The resulting slurry is diluted to 0.1 g/L, thereafter 10 ml of the resultant is collected and titrated with diallyldimethylammonium chloride (DADMAC) having a normality of 1/1000 by use of a streaming current detector (Mutek Particle Charge Detector 03), and the amount of DADMAC added until the streaming current is zero is used to calculate the degree of anionization according to the following expression:

$$q = (V \times c)/m$$

q: Degree of anionization (meq/g)
V: Amount (L) of DADMAC added until streaming current is zero
c: Concentration (meq/L) of DADMAC
m: Mass (g) of carboxymethylated pulp in measurement sample.

As can be seen from the above measurement method, the "degree of anionization" corresponds to the equivalent of DADMAC required for neutralization of an anionic group per unit mass of the carboxymethylated pulp, and also corresponds to the equivalent of anion per unit mass of the carboxymethylated pulp.

The carboxymethylated pulp preferably has a degree of anionization of 1.00 meq/g or less, preferably 0.00 meq/g or more and 1.00 meq/g or less, further preferably 0.00 meq/g or more and 0.80 meq/g or less, further preferably 0.00 meq/g or more and 0.60 meq/g or less. A carboxymethylated pulp having a degree of anionization in such a range is considered to have carboxymethyl groups not locally, but uniformly introduced into the entire cellulose and is considered to be able to further stably obtain a unique effect for the carboxymethylated pulp, for example, the effect of imparting shape retention ability and water absorption ability, as compared with a carboxymethylated pulp having a degree of anionization of more than 1.00 meq/g. The carboxymethylated pulp having such a degree of anionization can be produced by, for example, a method described below.

Carboxymethylated pulp can be generally produced by subjecting cellulose to an alkaline treatment (mercerization) and thereafter allowing the resulting mercerized cellulose (also referred to as "alkaline cellulose".) to react with a carboxymethylating agent (also referred to as "etherifying agent".).

The carboxymethylated pulp having a degree of carboxymethyl substitution of 0.50 or less and having a degree of crystallization of cellulose type I of 50% or more for use in the pulverized product and the additive of the present invention can be produced by, for example, without any limitation, mercerization (alkaline treatment of cellulose) in a solvent containing mainly water and thereafter carboxymethylation (also referred to as "etherification".) in a mixed solvent of water and an organic solvent. The carboxymethylated pulp thus obtained is homogeneous and stable in quality, is excellent in dispersion stability, is excellent in imparting of water retention ability and shape retention ability, and also has the characteristics of being relatively hardly made sticky even in contact with water and also hardly forming a clump in water, and is suitable for use as an additive, as compared with any carboxymethylated pulp obtained according to a conventional water mediated method (method including performing both mercerization and carboxymethylation in water as a solvent) or solvent mediated method (method including performing both mercerization and carboxymethylation in a solvent containing mainly an organic solvent). The above method has the advantage of achieving a high rate of effective utilization of a carboxymethylating agent.

First, the above described pulp is used as a raw material and a mercerizing agent (alkali) is added thereto, thereby obtaining mercerized pulp. Water can be mainly used in the solvent in the mercerization reaction and a mixed solvent of an organic solvent and water can be used in the next carboxymethylation, according to the method described herein, thereby obtaining carboxymethylated pulp suitable as the additive in an economic manner.

Such main use of water in the solvent (solvent containing mainly water) refers to a solvent containing water at a proportion of more than 50% by mass. The solvent containing mainly water preferably contains 55% by mass or more, more preferably 60% by mass or more, more preferably 70% by mass or more, more preferably 80% by mass or more, further preferably 90% by mass or more, further preferably 95% by mass or more of water. A particularly preferable solvent containing mainly water is a solvent containing 100% by mass of water (namely, water). A higher proportion of water in mercerization provides the advantage of more uniform introduction of carboxymethyl groups into cellulose. Examples of the solvent (which is mixed with water) other than water in the solvent containing mainly water include an organic solvent for use as a solvent in carboxymethylation at a later stage. Examples can include alcohols such as methanol, ethanol, n-propyl alcohol, isopropyl alcohol, n-butanol, isobutanol, and tert-butanol, ketones such as acetone, diethyl ketone, and methyl ethyl ketone, and dioxane, diethyl ether, benzene and dichloromethane, and these can be added singly or as a mixture of two or more thereof in an amount of less than 50% by mass, to water, and the resultant can be used as the solvent in mercerization. The solvent containing mainly water preferably contains 45% by mass or less, further preferably 40% by mass or less, further preferably 30% by mass or less, further preferably 20% by mass or less, further preferably 10% by mass or less, further preferably 5% by mass or less, more preferably 0% by mass of an organic solvent.

Examples of the mercerizing agent include alkali metal hydroxides such as lithium hydroxide, sodium hydroxide, and potassium hydroxide, and these can be used singly or in combinations of any two or more thereof. The mercerizing agent is not limited thereto, and such an alkali metal hydroxide can be added to a reactor, for example, in the form of an aqueous solution thereof, having, for example, a concentration of 1 to 60% by mass, preferably 2 to 45% by mass, more preferably 3 to 25% by mass.

The amount of the mercerizing agent used may be any amount and is not particularly limited as long as the carboxymethylated pulp can satisfy both a degree of carboxymethyl substitution of 0.50 or less and a degree of crystallization of cellulose type I of 50% or more, and, in one embodiment, the amount is preferably 0.1 mol or more and 2.5 mol or less, more preferably 0.3 mol or more and 2.0 mol or less, further preferably 0.4 mol or more and 1.5 mol or less based on 100 g (absolute dry) of cellulose.

The amount of the solvent containing mainly water in mercerization is preferably an amount which can allow for stirring and mixing of a raw material. Specifically, the amount, is preferably 1.5 to 20 times by mass, more preferably 2 to 10 times by mass, relative to a cellulose raw material, but is not limited thereto. Such an amount enables a homogeneous reaction to occur.

A mercerization treatment is performed by mixing a raw material (pulp) and the solvent containing mainly water, adjusting the temperature of a reactor to 0 to 70°C, preferably 10 to 60°C, more preferably 10 to 40°C, adding an aqueous mercerizing agent solution, and stirring the resultant for 15 minutes to 8 hours, preferably 30 minutes to 7 hours, more preferably 30 minutes to 3 hours. Thus, the mercerized pulp is obtained.

The pH in mercerization is preferably 9 or more, and thus the mercerization reaction can progress. The pH is more preferably 11 or more, further preferably 12 or more, and may be 13 or more. The upper limit of the pH is not particularly limited.

Such mercerization can be performed by use of a reaction machine in which the respective components can be mixed and stirred with the temperature being controlled, and any of various reaction machines conventionally used in a mercerization reaction can be used. For example, a batch type stirring apparatus in which the respective components are mixed under stirring with two screws is preferable from both viewpoints of uniform mixing ability and productivity.

Next, a carboxymethylating agent (also referred to as "etherifying agent".) is added to the mercerized pulp, thereby obtaining carboxymethylated pulp. The solvent containing mainly water can be used in mercerization and the mixed solvent of water and an organic solvent can be used in carboxymethylation, according to the method described herein, thereby obtaining carboxymethylated pulp suitable as the above additive, in an economic manner.

Examples of the carboxymethylation agent include monochloroacetic acid, sodium monochloroacetate, methyl monochloroacetate, ethyl monochloroacetate, and isopropyl monochloroacetate. In particular, monochloroacetic acid or sodium monochloroacetate is preferable in terms of their availability.

The amount of the carboxymethylating agent used may be any amount and is not particularly limited as long as the carboxymethylated pulp can satisfy both a degree of carboxymethyl substitution of 0.50 or less and a degree of crystallization of cellulose type I of 50% or more, and, in one embodiment, the carboxymethylating agent is preferably added in the range from 0.5 to 1.5 mol per anhydrous glucose unit of cellulose. The above range more preferably has a lower limit of 0.6 mol or more, further preferably 0.7 mol or more, and more preferably has an upper limit of 1.3 mol or less, further preferably 1.1 mol or less. The carboxymethylating agent, but is not limited to, can be added to a reactor, in the form of an aqueous solution having, for example, a concentration of 5 to 80% by mass, more preferably 30 to 60% by mass, or can be added in the form of a powder with being not dissolved.

The molar ratio of the mercerizing agent to the carboxymethylating agent (mercerizing agent/carboxymethylating agent), generally adopted, is 0.90 to 2.45 in a case where monochloroacetic acid or sodium monochloroacetate is used as the carboxymethylating agent. The reason for this is because a molar ratio of less than 0.90 can cause a carboxymethylation reaction to be insufficient, resulting in remaining of the unreacted monochloroacetic acid or sodium monochloroacetate and thus diseconomy, and a molar ratio of more than 2.45 can cause a side reaction of an excess of the mercerizing agent with monochloroacetic acid or sodium monochloroacetate to progress, probably resulting in production of a glycolic acid alkali metal salt and thus the risk of diseconomy.

The rate of effective utilization of the carboxymethylating agent in carboxymethylation is preferably 15% or more, more preferably 20% or more, further preferably 25% or more, particularly preferably 30% or more. The rate of effective utilization of the carboxymethylating agent refers to the proportion of carboxymethyl groups introduced into cellulose in carboxymethyl groups in the carboxymethylating agent. The solvent containing mainly water can be used in mercerization and the mixed solvent of water and an organic solvent can be used in carboxymethylation, thereby producing the carboxymethylated pulp in the present invention at a high rate of effective utilization of the carboxymethylating agent (namely, in an economic manner without use of large amount of the carboxymethylating agent). The upper limit of the rate of effective utilization of the carboxymethylating agent is not particularly limited, and the lower limit is actually about 80%. The rate of effective utilization of the carboxymethylating agent may be here abbreviated as AM

The method for calculating the rate of effective utilization of the carboxymethylating agent is as follows:

$$AM = (DS \times \text{Number of moles of cellulose})/\text{Number of moles of carboxymethylating agent}$$

DS: Degree of carboxymethyl substitution
Number of moles of cellulose: Mass of pulp (Dry mass after drying at 100°C for 60 minutes)/162
(162 means the molecular weight per glucose unit of cellulose).

The concentration of the pulp in the carboxymethylation reaction is not particularly limited, and is preferably 1 to 40% (w/v) from the viewpoint of an increase in rate of effective utilization of the carboxymethylating agent.

The carboxymethylation reaction is allowed to progress in a mixed solvent of water and an organic solvent, the mixed solvent of water and an organic solvent being formed by appropriately adding an organic solvent or an aqueous solution of an organic solvent to a reactor or appropriately decreasing the organic solvent or the like other than water in the mercerization treatment, for example, under reduced pressure, at the same time as addition of the carboxymethylating agent or before or immediately after addition of the carboxymethylating agent. The timing of addition or decrease of the organic solvent may be any timing and is not particularly limited as long as it is within the time from completion of the mercerization reaction to the time immediately after addition of the carboxymethylating agent, and is preferably, for example, within 30 minutes before or after addition of the carboxymethylating agent.

Examples of the organic solvent can include alcohols such as methanol, ethanol, n-propyl alcohol, isopropyl alcohol, n-butanol, isobutanol, and tert-butanol, ketones such as acetone, diethyl ketone, and methyl ethyl ketone, and dioxane, diethyl ether, benzene and dichloromethane, and these can be added singly or as a mixture of two or more thereof, to water, and the resultant can be used as the solvent in carboxymethylation. In particular, a monohydric alcohol having 1 to 4 carbon atoms is preferable and a monohydric alcohol having 1 to 3 carbon atoms is further preferable, because of being excellent in compatibility with water.

The proportion of the organic solvent in the mixed solvent in carboxymethylation is preferably 20% by mass or more, more preferably 30% by mass or more, further preferably 40% by mass or more, further preferably 45% by mass or more, particularly preferably 50% by mass or more, based on the total of water and the organic solvent. As the proportion of the organic solvent is higher, more uniform substitution of carboxymethyl groups occurs, and, for example, an advantage is that carboxymethylated pulp which is homogeneous and stable in quality is obtained. The upper limit of the proportion of the organic solvent is not limited, and may be, for example, 99% by mass or less. The upper limit is preferably 90% by mass or less, further preferably 85% by mass or less, further preferably 80% by mass or less, further preferably 70% by mass or less in consideration of the cost of the organic solvent added.

The reaction medium (the mixed solvent of, water and the organic solvent, containing no pulp) in carboxymethylation is preferably lower in proportion of water than the reaction medium in mercerization (in other words, higher in proportion of the organic solvent). Such a range can be satisfied to thereby allow the degree of crystallization of the resulting carboxymethylated pulp to be easily maintained and allow the carboxymethylated pulp in the present invention to be more efficiently obtained. In a case where the reaction medium in carboxymethylation is lower in proportion of water than the reaction medium in mercerization (higher in proportion of the organic solvent), an advantage is that the mixed solvent for the carboxymethylation reaction can be obtained by a simple procedure in which a desired amount of the organic solvent is added to the reaction system after completion of the mercerization reaction, in transferring from the mercerization reaction to the carboxymethylation reaction.

After the mixed solvent of water and an organic solvent is formed and the carboxymethylating agent is added to the mercerized pulp, the resultant is stirred for 15 minutes to 4 hours, preferably about 15 minutes to 1 hour with the temperature being preferably kept constantly in the range from 10 to 40°C. A liquid containing the mercerized pulp is preferably mixed with the carboxymethylating agent in portions or by dropping in order to prevent the reaction mixture from being at a high temperature. After the carboxymethylating agent is added and stirred for a certain time, the temperature is, if necessary, raised so that the reaction temperature is 30 to 90°C, preferably 40 to 90°C, further preferably 60 to 80°C. An etherification (carboxymethylation) reaction is performed for 30 minutes to 10 hours, preferably 1 hour to 4 hours, thereby obtaining carboxymethylated pulp. An advantage is that such a temperature rise in the carboxymethylation reaction allows the etherification reaction to be efficiently performed in a short time.

The reactor used in carboxymethylation may be the reactor used in mercerization or another reactor in which the respective components can be mixed and stirred with the temperature being controlled.

After completion of the reaction, the remaining alkali metal salt may be neutralized with a mineral acid or organic acid. If necessary, an inorganic salt, an organic acid salt, and/or the like as by-product(s) may be removed by washing with water-containing methanol, and the resultant may be dried, pulverized and classified to form the carboxymethylated pulp or the salt thereof. The drying method is not limited at all, and, for example, a known method can be used such as a freeze-drying method, a spray-drying method, a shelf-type drying method, a drum drying method, a belt drying method, a drying method including thinly extending on a glass plate or the like, a fluid-bed drying method, a microwave drying method, or a drying method including using a heat generating fan under reduced pressure. The pulverizing method is also not particularly limited, the apparatus for use in dry pulverization can be, for example, an impact mill such as a hammer mill or a pin mill, a medium mill such as a ball mill or a tower mill, or a jet mill, and the apparatus for use in wet pulverization can be, for example, a homogenizer, a masscolloider, or a pearl mill.

In a case where the carboxymethylated pulp for use in the pulverized product and the additive of the present invention is produced, the pulp as a raw material or the pulp after carboxymethylation may be, if necessary, subjected to acid hydrolysis with mineral acid such as hydrochloric acid, sulfuric acid, or nitric acid. The carboxymethylated pulp that has been subjected to acid hydrolysis can be used as a raw material of powdery cellulose, and such powdery cellulose may also be, if necessary, further subjected to a neutralization, drying, pulverization, or classification treatment.

The above production method can provide favorable carboxymethylated pulp which is homogeneous, and achieves good water retention ability and shape retention ability, although the carboxymethylated pulp has a degree of carboxymethyl substitution of 0.50 or less, has a degree of crystallization of cellulose type I of 50% or more. It is unclear why such carboxymethylated pulp can be obtained by the above method. However, the present inventors presume the following:

the mercerization reaction is performed using the solvent containing mainly water to thereby not only allow the mercerizing agent to be uniformly admixed, but also allow the mercerization reaction to more uniformly occur, and the organic solvent is present during carboxymethylation to thereby enhance the rate of effective utilization of the carboxymethylating agent, resulting in less occurrence of a side reaction by an excess of the carboxymethylating agent (for example, production of a glycolic acid alkali metal salt) and stabilization of quality. Thus, carboxymethylation uniformly occurs and the carboxymethylated pulp is easily uniformly dispersed, resulting in a reduction in proportion of a filtration residue generated. However, there is not limited thereto.

<Pulverized product of carboxymethylated pulp>

The pulverized product of carboxymethylated pulp can be obtained by the above dry pulverization or wet pulverization of carboxymethylated pulp. Among the dry pulverization and wet pulverization, the wet pulverization is more preferable. Further, fibers of the carboxymethylated pulp are preferably fibrillated by pulverization.

Carboxymethylated pulp fibrillated by pulverization is obtained by properly beating or defibrating (fibrillating) carboxymethylated pulp with a refiner or the like. In a pulverized product of the fibrillated carboxymethylated pulp, fluff of microfibril of cellulose is observed on a fiber surface, as compared with a pulverized product of carboxymethylated pulp not beaten or defibrated. Such a pulverized product of the fibrillated carboxymethylated pulp has a shape having a larger fiber diameter and including a fiber surface efficiently fluffed (externally fibrillated) with a fiber itself being inhibited from being finer (fiber inhibited from internally fibrillated), as compared with a carboxymethylated cellulose nanofiber (hereinafter, also sometimes abbreviated as "CNF".).

The pulverized product of the fibrillated carboxymethylated pulp has carboxymethyl groups and thus has the characteristics of high water retention ability and thixotropic properties, etc. as compared with a fibrillated pulp that is not carboxymethylated.

The pulverized product of the fibrillated carboxymethylated pulp has carboxymethyl groups in the pulp during fibrillation and thus has the characteristics that hydrogen linkages between fibers is weakened due to introduction of carboxymethyl groups, to thereby allow fibers to easily loosen in fibrillation, resulting in less damage of fibers, as compared with one obtained by first beating a pulp that is not carboxymethylated and then carboxymethylating the beaten pulp.

Such defibrating or beating in fibrillation of carboxymethylated pulp is preferably performed in a wet manner (namely, in a mode of a dispersion using water or the like as a dispersing medium) by use of, for example, without particular limitation, a disc, conical, or cylindrical refiner, high-speed defibrating machine, shear stirring machine, colloid mill, high-pressure spray dispersing machine, beater, PFI mill, kneader, or disperser, but any apparatus may be used which imparts a mechanical defibrating force in a wet manner.

The raw material of a dispersion of carboxymethylated pulp to be subjected to fibrillation preferably has a solid content concentration of 0.1% by mass or more, further preferably 0.5% by mass or more, further preferably 1.0% by mass or more, further preferably 2.0% by mass or more. The carboxymethylated pulp having a specified degree of carboxymethyl substitution and a specified degree of crystallization, for use in the pulverized product of the present invention, has the characteristics of being less in stickiness, and hardly causing the problem of, for example, clogging in an apparatus even when used at a relatively high concentration. The upper limit of the concentration is preferably 40% by mass or less, further preferably 30% by mass or less.

The carboxymethylated pulp obtained by the above method may be dried and pulverized in advance before preparation of the dispersion to be subjected to fibrillation. Next, the carboxymethylated pulp dry pulverized may be dispersed in a dispersing medium and subjected to (wet) fibrillation. The apparatus for use in dry pulverization of the raw material is not particularly limited, and examples thereof can include impact mills such as a hammer mill and a pin mill, medium mills such as a ball mill and a tower mill, and jet mills.

The pulverized product of carboxymethylated pulp or the pulverized product of the fibrillated carboxymethylated pulp (hereinafter, collectively called "pulverized product".) preferably has an average fiber diameter of 500 nm or more, preferably more than 500 nm, further preferably 1 $\mu$m or more, more preferably 10 $\mu$m or more. The upper limit of the average fiber diameter is preferably 60 $\mu$m or less, more preferably 40 $\mu$m or less, further preferably 30 $\mu$m or less, further preferably 20 $\mu$m or less. Proper pulverization or fibrillation so that the average fiber diameter is in the above range allows the pulverized product to exhibit higher water retention ability, as compared with a cellulose fiber that is not defibrated, and to achieve higher effects of imparting strength and enhancing yield even in a small amount thereof, as compared with a finely defibrated cellulose nanofiber.

The pulverized product preferably has an average fiber length of 200 $\mu$m or more, preferably 300 $\mu$m or more, more preferably 500 $\mu$m or more. The upper limit of the average fiber length is not particularly limited, and is preferably 3000 $\mu$m or less, preferably 1500 $\mu$m or less, further preferably 1100 $\mu$m or less, further preferably 900 $\mu$m or less. According to the present invention, carboxymethylated pulp is used for pulverization such as beating or defibrating, and thus fibrillation can progress without any extreme fiber shortening.

The average fiber diameter and the average fiber length can be determined by a fiber analyzer with image analysis, for example, L&W Fiber Tester Plus manufactured by ABB or a fractionator manufactured by Valmet. Specifically, the diameter and the length can be determined as the length-weighted fiber width and the length-weighted average fiber length, respectively, in the case of use of such a fractionator.

The pulverized product preferably has an aspect ratio of 10 or more, more preferably 20 or more, further preferably 30 or more. The upper limit of the aspect ratio is not particularly limited, and is preferably 1000 or less, more preferably 100 or less, further preferably 80 or less. The aspect ratio can be calculated by the following expression:
Aspect ratio = Average fiber length/Average fiber diameter.

The pulverized product of the fibrillated carboxymethylated pulp preferably has a rate of fibrillation (Fibrillation %) of 1.0% or more, more preferably 2.5% or more, further preferably 3.5% or more, as measured with a fractionator manufactured by Valmet. The rate of fibrillation corresponds to the ratio of a fibril area to the total of a fiber area and the fibril area. The rate of fibrillation varies depending on the type of a cellulose raw material used. If the rate is in the above range, it is considered that cellulose material is fibrillated. In the present invention, fibrillation is preferably performed so as to enhance the rate of fibrillation (fo) of the fibrillated carboxymethylated pulp as compared to the carboxymethylated pulp before fibrillation. In a case where the rate of fibrillation of the pulverized product of the fibrillated carboxymethylated pulp is designated as "f", the difference in rate of fibrillation ($\Delta f = f - f_0$) may be more than 0, and is preferably 0.2% or more, more preferably 1% or more, further preferably 2.5% or more.

The degree of carboxymethyl substitution of the pulverized product is usually the same as the degree of carboxymethyl substitution of carboxymethylated pulp before pulverization and/or fibrillation.

The pulverized product preferably has a degree of crystallization of cellulose type I of 50% or more, more preferably 60% or more. The upper limit of the degree of crystallization of cellulose type I is not particularly limited. The upper limit is considered to be actually about 90%. The method for measuring the degree of crystallization of cellulose type I is as described above.

The pulverized product preferably has a degree of anionization (also referred to as "density of anionic charge".) of 0.10 meq/g or more and 2.00 meq/g or less. The method for measuring the degree of anionization of the pulverized product is as follows:

The pulverized product is dispersed in water to prepare a water dispersion having a solid content of 10 g/L, and the water dispersion is stirred with a magnetic stirrer at 1000 rpm for 10 minutes or more. The resulting slurry is diluted to 0.1 g/L, thereafter 10 ml of the resultant is collected and titrated with diallyldimethylammonium chloride (DADMAC) having a normality of 1/1000 by use of a streaming current detector (Mutek Particle Charge Detector 03), and the amount of DADMAC added until the streaming current is zero is used to calculate the degree of anionization according to the following expression:

$$q = (V \times c)/m$$

q: Degree of anionization (meq/g)
V: Amount (L) of DADMAC added until streaming current is zero
c: Concentration (meq/L) of DADMAC
m: Mass (g) of carboxymethylated pulp in measurement sample.

The pulverized product preferably has a degree of anionization of 0.10 meq/g or more and 2.00 meq/g or less, more preferably 0.10 meq/g or more and 1.50 meq/g or less, further preferably 0.10 meq/g or more and 1.30 meq/g or less, further preferably 0.10 meq/g or more and 1.00 meq/g or less, further preferably 0.10 meq/g or more and 0.80 meq/g or less. A pulverized product having a degree of anionization in such a range is considered to have carboxymethyl groups not locally, but uniformly introduced into the entire cellulose and is considered to be able to further stably obtain the effect of imparting water retention ability, as compared with a carboxymethylated pulp high in degree of anionization.

The pulverized product has the characteristics of exhibiting a relatively low viscosity when in the form of a dispersion using water as a dispersing medium (water dispersion). The method for measuring the viscosity of the pulverized product is as follows:

The pulverized product is metered and taken in a polypropylene container and dispersed in 160 ml of ion-exchange water, thereby preparing a water dispersion so that the solid content is 1% by mass. The temperature of the water dispersion is adjusted to 25°C. Thereafter, the viscosity at a rotational speed of 60 rpm after 1 minute is measured with a B-type viscometer (manufactured by Toki Sangyo Co., Ltd.) according to the method of JIS-Z-8803.

The pulverized product preferably has a viscosity (25°C, 60 rpm) of 2500 mPa•s or less. The lower limit value is preferably 10 mPa•s or more, more preferably 20 mPa•s or more, further preferably 50 mPa•s or more. The upper limit value is more preferably 2000 mPa•s or less, further preferably 1500 mPa•s or less, further preferably 1000 mPa•s or less, further preferably 600 mPa•s or less, further preferably 300 mPa•s or less.

The pulverized product preferably has a water retention capacity of 15 or more, as measured according to the following method. The method for measuring the water retention capacity is as follows:

Forty mL of a slurry (medium: water) of the pulverized product, having a solid content of 0.3% by mass, is prepared. The mass of the slurry is designated as "A". Next, the total amount of the slurry is centrifuged by a high-speed cooling centrifuge at 30°C and 25000 G for 30 minutes, and an aqueous phase and a sediment are separated. The mass of the sediment is designated as "B". The aqueous phase is placed in an aluminum cup and dried at 105°C all night and all day, thereby removing water, and the mass of the solid content in the aqueous phase is measured. The mass of the solid content in the aqueous phase is designated as "C". The water retention capacity is calculated according to the following expression:

$$\text{Water retention capacity} = (B + C - 0.003 \times A)/(0.003 \times A - C).$$

The water retention capacity corresponds to the mass of water in the sediment relative to the mass of the solid content of the fiber in the sediment, as represented by the above expression. A larger value means that a fiber has a higher water retention ability. The pulverized product preferably has a water retention capacity of 15 or more, more preferably 20 or more, further preferably 30 or more. The upper limit is not particularly limited, and considered to be actually about 200 or less.

The method for measuring the water retention capacity is directed to the pulverized product of the fibrillated carboxymethylated pulp, and cannot be usually applied to a fiber that is not fibrillated or defibrated, nor CNF finely defibrated. If the water retention capacity of a cellulose fiber that is not fibrillated or defibrated is tried to be measured according to the above method, no dense sediment can be formed in the above centrifugation conditions and the sediment and the aqueous phase are difficult to separate. CNF is almost not sedimented in the above centrifugation conditions.

The pulverized product, which is in the form of a water dispersion having a solid content concentration of 1.0% by mass, preferably has an electrical conductivity of 500 mS/m or less, more preferably 300 mS/m or less, further preferably 200 mS/m or less, further preferably 100 mS/m or less, further preferably 70 mS/m or less. The lower limit of the electrical conductivity is preferably 5 mS/m or more, more preferably 10 mS/m or more. The electrical conductivity of the pulverized product can be measured according to the following method:

200 g of a water dispersion of the pulverized product having a solid content concentration of 1.0% by mass is prepared and sufficiently stirred. Thereafter, the electrical conductivity is measured with an electrical conductivity meter (ES-71 Model manufactured by HORIBA Ltd.).

The pulverized product preferably has a BET specific surface area of 30 $m^2/g$ or more, more preferably 50 $m^2/g$ or more, further preferably 100 $m^2/g$ or more. The pulverized product, which has a large BET specific surface area, has the advantage of, for example, being easily bonded to pulp to result in an enhancement in yield and an improvement in effect of imparting strength to paper, when used as an additive for papermaking. The BET specific surface area of the pulverized product can be measured according to the following method with reference to a nitrogen gas adsorption method (JIS Z 8830):

(1) About 2% of a slurry of the pulverized product (dispersing medium: water) is taken so that the solid content is about 0.1 g, and placed in a centrifuge container, and 100 ml of ethanol is added thereto.
(2) A stirring bar is placed thereinto, and used for stirring at 500 rpm for 30 minutes.
(3) The stirring bar is taken out, and pulp is sedimented by a centrifuge machine in conditions of 7000 G, 30 minutes, and 30°C.
(4) The supernatant is removed while the sedimented pulp is not removed as much as possible.
(5) Addition of 100 ml of ethanol, addition of a stirring bar, stirring in the conditions in (2), centrifugation in the conditions in (3), and removal of the supernatant in the conditions in (4) are made, and repeated three times.
(6) The solvent in (5) is changed from ethanol to t-butanol, and the same stirring, centrifugation, and removal of the supernatant, as in (5), are repeated three times at room temperature equal to or more than the melting point of t-butanol.
(7) After the last removal of the solvent, 30 ml of t-butanol is added thereto and slightly admixed, and thereafter the resultant is transferred to a round-bottom flask and frozen with an ice bath.
(8) Cooling is made in a freezer for 30 minutes or more.
(9) The resultant is placed in a freeze-dryer, and freeze-drying is conducted for 3 days.
(10) BET measurement is performed (pre-treatment conditions: 105°C/2 hours in a nitrogen stream, relative pressure: 0.01 to 0.30, and amount of a sample: about 30 mg).

The pulverized product preferably has a Schopper-Riegler freeness of 1°SR or more, more preferably 10°SR or more, more preferably 25°SR or more. The method for measuring the Schopper-Riegler freeness of the pulverized product is according to JIS P 82121-1: 2012, and is specifically as follows:

The pulverized product is dispersed in water to prepare a water dispersion having a solid content of 10 g/L, and the water dispersion is stirred with a magnetic stirrer at 1000 rpm all night and all day. The resulting slurry is diluted to 1 g/L. A 60-mesh screen (wire diameter: 0.17 mm) is installed to DFR-04 manufactured by Mutec Co., Ltd., the amount of a liquid passing through the mesh, in 1000 ml of a testing liquid, is measured for 60 seconds, and the Schopper-Riegler freeness is calculated by the method according to JIS P 8121-1: 2012.

The Schopper-Riegler freeness of the pulverized product is not particularly limited, and the lower limit thereof is preferably 1°SR or more, more preferably 10°SR or more, more preferably 25°SR or more, more preferably 40°SR or more, further preferably 50°SR or more. The upper limit is not particularly limited, and is 100°SR or less.

The pulverized product, which is in the form of a water dispersion having a solid content of 1% by mass, preferably has a transparency (transmittance of light at 660 nm) of less than 60%, further preferably 40% or less, further preferably 30% or less, further preferably 20% or less, further preferably 10% or less. The lower limit is not particularly limited, and may be 0% or more. A transparency in such a range provides a proper degree of fibrillation and allows the effects of the present invention to be easily obtained. The transparency of the pulverized product can be measured according to the following method:

A water dispersion (solid content: 1% (w/v), dispersing medium: water) of the pulverized product is prepared, and the transmittance of light at a wavelength of 660 nm is measured with an UV-VIS spectrophotometer UV-1800 (manufactured by Shimadzu Corporation) and a square cell having a length of light path of 10 mm.

The pulverized product in the form of a water dispersion having a solid content concentration of about 2% or more becomes translucent to white gel, cream, or paste.

The pulverized product may be in the form of a dispersion obtained after production, and may be, if necessary, dried or re-dispersed in water. The drying method is not limited at all, and any known method such as a freeze-drying method, a spray-drying method, a shelf-type drying method, a drum drying method, a belt drying method, a drying method including thinly extending on a glass plate or the like, a fluid-bed drying method, a microwave drying method, or a drying method including using a heat generating fan under reduced pressure can be used. After drying, the resultant may be, if necessary, pulverized by a cutter mill, a hammer mill, a pin mill, a j et mill, or the like. The method for re-dispersing in water is also not particularly limited, and any known dispersing apparatus can be used.

<Additive>

A pulverized product having a degree of carboxymethyl substitution of 0.50 or less and having a degree of crystallization of cellulose type I of 50% or more, obtained according to the above production method, is homogeneous and excellent in dispersion stability, is excellent in imparting of water retention ability and shape retention ability, is relatively less sticky even in contact with water, and hardly forms a clump (aggregate) in water, and therefore can be suitably used for various additives such as an agent imparting water retention ability, an agent imparting shape retention ability, a viscosity modifier, an emulsion stabilizer, and a dispersion stabilizer in various fields including food products, pharmaceutical products, cosmetic products, feeds, papermaking, paints, and the like.

The additive in the present invention can be used in various fields in which an additive is generally used, and can be used as, for example, a thickener, a gelling agent, a pasting agent, a food additive, an excipient, an additive for paints, an additive for adhesives, an additive for papermaking, a polishing agent, a compounding material for rubber or plastics, an agent imparting water retention ability, an agent imparting shape retention ability, a viscosity modifier, an emulsion stabilizer, a dispersion stabilizer, a mud adjuster, a filtration aid, a mud overflow inhibitor, or the like in, for example, without any limitation, food products, beverages, cosmetic products, pharmaceutical products, papermaking, various chemical goods, paints, spray, feeds, agricultural chemicals, civil engineering, architecture, electronic materials, flame retardants, household products, adhesives, detergents, aromatic substances, and lubricant compositions.

Examples of the additive for food products include, but not limited to, an agent imparting water retention ability, an agent imparting shape retention ability, a viscosity modifier, an emulsion stabilizer, and a dispersion stabilizer, which are for use in food products. Examples of any usable food product include, but not limited to, beverages (cocoa, fiber/pulp-containing juice, sweet red-bean soup, amazake, probiotic drinks, fruit milk, and the like), soups (corn soup, ramen soup, miso soup, consomme, and the like), sauces, dressing, ketchup, mayonnaise, jam, yogurt, whip cream, dry foods (dry processed food, instant noodle, pasta noodle, and the like), gluten-free pasta, ice cream, monaka (bean-jam-filled wafers), sherbet, polyjuice, confectionery (gummi candy, soft candy, jelly, cookie, and the like), merengue, breads (sweet bun, custard cream bread, and the like), gluten-free breads, fillings, pancakes, pastes, and edible films.

Examples of the additive for pharmaceutical products include, but not limited to, an agent imparting water retention ability, an agent imparting shape retention ability, a viscosity modifier, an emulsion stabilizer, and a dispersion stabilizer, which are for use in pharmaceutical products. Examples of any usable food product include, but not limited to, a tablet, an ointment, an adhesive tape, a poultice, a hand cream, and a toothpaste.

Examples of the additive for cosmetic products include, but not limited to, an agent imparting water retention ability, an agent imparting shape retention ability, a viscosity modifier, an emulsion stabilizer, and a dispersion stabilizer, which are for use in cosmetic products. Examples of such cosmetic products include a face powder, a foundation, a scrub agent for face washing, a pack, a cleansing foam, a cleansing cream, a hair mousse, a shampoo, soap, a lotion, a hair color, a hair bleaching agent, mascara, an eyeliner, a manicure, and an antiperspirant.

Examples of the additive for feeds include, but not limited to, an agent imparting water retention ability, an agent imparting shape retention ability, a viscosity modifier, an emulsion stabilizer, and a dispersion stabilizer, which are for use in feeds. Examples of such feeds include a moist pellet and an expansion pellet for domestic animals or cultured fishes, and a milk substitute for cattle.

Examples of the additive for papermaking include, but not limited to, an agent imparting water retention ability, an agent imparting shape retention ability, a viscosity modifier, an emulsion stabilizer, and a dispersion stabilizer, which are for use in papermaking. For example, such an additive can be used as, for example, a surface sizing agent, a retention aid, a paper strengthening agent, a coating agent, or an agent for a bulky paper.

Examples of the additive for paints include, but not limited to, an agent imparting water retention ability, an agent imparting shape retention ability, a viscosity modifier, an emulsion stabilizer, and a dispersion stabilizer, which are for use in paints. Examples of such paints include a matte paint, a paint for building, and an automobile interior paint.

Such an additive can also be used in, for example, filtration (moisture removal) of edible oil or various solvents; a building material such as a fiber wall, a sand wall, or a gypsum board; civil engineering such as foam shielding or a water sealant for a continuous wall; a resin filler or a compound such as foamed polystyrene, a biodegradable resin, rubber, ceramic, or vinyl chloride; a dispersant for dispersing, such as fine particle carbon black, barium sulfate (X-ray contrast agent), titanium oxide, or zing oxide; a moisture absorbent aid for an improvement in shape retention ability in moisture absorption, for example, a deliquescent agent such as calcium chloride; a modifier for fibers (cloth, yarn); a liquid carrier; a lubricating oil; ceramic engineering; cat sand; a water absorption material for a desiccant; a greening construction method; or a binder.

EXAMPLES

Hereinafter, the present invention will be more specifically described with reference to Examples and Comparative Examples, but the present invention is not limited thereto. Unless especially noted, "part(s)" and "%" represent "part(s) by mass" and "% by mass", respectively.

(Production Example 1 of carboxymethylated pulp)

To a twin-screw kneader whose rotational speed was modulated to 100 rpm, were added 130 parts of water and a solution of 20 parts of sodium hydroxide in 100 parts of water, and hardwood pulp (manufactured by Nippon Paper Industries Co., Ltd., LBKP) was added thereto in an amount of 100 parts in terms of dry mass in drying at 100°C for 60 minutes. The resultant was stirred and mixed at 30°C for 90 minutes, thereby preparing a cellulose raw material mercerized. After 100 parts of isopropanol (IPA) and 60 parts of sodium monochloroacetate were added with further stirring and the resultant was stirred for 30 minutes, the temperature was raised to 70°C, thereby allowing for a carboxymethylation reaction for 90 minutes. The concentration of IPA in the reaction medium in the carboxymethylation reaction was 30%. After completion of the reaction, the resultant was neutralized with acetic acid to a pH of about 7, and thereafter subjected to liquid removal and drying, thereby obtaining carboxymethylated pulp having a degree of carboxymethyl substitution of 0.21 and a degree of crystallization of cellulose type I of 72%. The rate of effective utilization of the carboxymethylating agent was 29%. The methods for measuring the degree of carboxymethyl substitution and the degree of crystallization of cellulose type I, and the method for calculating the rate of effective utilization of the carboxymethylating agent are as described above.

(Production Example 2 of carboxymethylated pulp)

To a twin-screw kneader whose rotational speed was modulated to 100 rpm, were added 125 parts of water and a solution of 20 parts of sodium hydroxide in 100 parts of water, and hardwood pulp (manufactured by Nippon Paper Industries Co., Ltd., LBKP) was added thereto in an amount of 100 parts in terms of dry mass in drying at 100°C for 60 minutes. The resultant was stirred and mixed at 30°C for 90 minutes, thereby preparing a cellulose raw material mercerized. After 100 parts of isopropanol (IPA) and 60 parts of sodium monochloroacetate were added with further stirring and the resultant was stirred for 30 minutes, the temperature was raised to 70°C, thereby allowing for a carboxymethylation reaction for 90 minutes. The concentration of IPA in the reaction medium in the carboxymethylation reaction was 31%. After completion of the reaction, the resultant was neutralized with acetic acid to a pH of about 7, and thereafter subjected to liquid removal, drying, and pulverization, thereby obtaining carboxymethylated pulp having a degree of carboxymethyl substitution of 0.25 and a degree of crystallization of cellulose type I of 74%.
The rate of effective utilization of the carboxymethylating agent was 30%.

(Production Example 3 of carboxymethylated pulp)

To a twin-screw kneader whose rotational speed was modulated to 100 rpm, were added 75 parts of water and a solution of 20 parts of sodium hydroxide in 100 parts of water, and hardwood pulp (manufactured by Nippon Paper Industries Co., Ltd., LBKP) was added thereto in an amount of 100 parts in terms of dry mass in drying at 100°C for 60 minutes. The resultant was stirred and mixed at 30°C for 90 minutes, thereby preparing a cellulose raw material mercerized. After 100 parts of isopropanol (IPA) and 60 parts of sodium monochloroacetate were added with further stirring and the resultant was stirred for 30 minutes, the temperature was raised to 70°C, thereby allowing for a carboxymethylation reaction for 90 minutes. The concentration of IPA in the reaction medium in the carboxymethylation reaction was 37%. After completion of the reaction, the resultant was neutralized with acetic acid to a pH of about 7, and thereafter subjected to liquid removal and drying, thereby obtaining carboxymethylated pulp having a degree of carboxymethyl substitution of 0.38 and a degree of crystallization of cellulose type I of 59%. The rate of effective utilization of the carboxymethylating agent was 46%.

(Example 1)

A water dispersion of the carboxymethylated pulp obtained in Production Example 1 having a solid content concentration of 4% by mass was prepared, and treated with Laboratory Refiner manufactured by Aikawa Iron Works Co., Ltd. for 10 minutes, thereby preparing a pulverized product of fibrillated carboxymethylated pulp. The resulting pulverized product was subjected to measurement to provide respective values of physical properties described in Table 1. The methods for measuring the respective values of physical properties are as described in the section "Pulverized product of carboxymethylated pulp" as the measurement methods of the pulverized product. The results are shown in Table 1.

(Example 2)

A pulverized product of fibrillated carboxymethylated pulp was prepared in the same manner as in Example 1 except that the solid content concentration of the carboxymethylated pulp obtained in Production Example 1 was changed from 4% by mass to 2% by mass and TopFiner manufactured by Aikawa Iron Works Co., Ltd. was used. The results are shown in Table 1.

(Example 3)

A pulverized product of fibrillated carboxymethylated pulp was prepared in the same manner as in Example 2 except that a water dispersion of the carboxymethylated pulp obtained in Production Example 2, having a solid content concentration of 4% by mass, was used. The results are shown in Table 1.

(Example 4)

A pulverized product of fibrillated carboxymethylated pulp was prepared in the same manner as in Example 2 except that a water dispersion of the carboxymethylated pulp obtained in Production Example 3 having a solid content concentration of 4% by mass was used. The results are shown in Table 1.

(Comparative Example 1)

A water dispersion of softwood pulp (NBKP manufactured by Nippon Paper Industries Co., Ltd.) having a solid content concentration of 4% by mass was prepared, and treated with TopFiner manufactured by Aikawa Iron Works Co., Ltd. for 10 minutes, thereby preparing fibrillated pulp. The results are shown in Table 1.

[Table 1]

| | Raw material used | Pulverization equipment | DS | Degree of crystallization (%) | Average fiber length (mm) | Average fiber diameter ($\mu$m) | Degree of anionization (meq/g) | Viscosity at 1% and 60 rpm (mPa·s) | Water retention capacity | Electrical conductivity (mS/m) | BET specific surface area (m²/g) | Aspect ratio |
|---|---|---|---|---|---|---|---|---|---|---|---|---|
| Example 1 | Production Example 1 | LCR | 0.21 | 72.3 | 0.37 | 13.8 | 0.58 | 413 | 77 | 52 | 178 | 27 |
| Example 2 | Production Example 1 | TF | 0.21 | 69.5 | 0.54 | 13.8 | 0.61 | 1580 | 120 | 55 | 275 | 39 |
| Example 3 | Production Example 2 | TF | 0.25 | 74.1 | 0.35 | 15.7 | 0.53 | 79 | 51 | 42 | 188 | 22 |
| Example 4 | Production Example 3 | TF | 0.38 | 59.0 | 0.46 | 13.4 | 1.09 | 840 | 98 | 90 | 274 | 34 |
| Comparative Example 1 | NBKP | TF | 0 | 64 | 1.73 | 16.3 | 0.05 | 125 | 13 | 5 | Not measured | 106 |

\* LCR: Laboratory Refiner
\* TF: TopFiner

It was found from the results in Table 1 that each of the pulverized products of fibrillated carboxymethylated pulp (Examples 1 to 4), according to the present invention, was high in water retention capacity as compared with that of Comparative Example 1 which was not carboxymethylated. The pulp (Examples 1 to 4), according to the present invention, hardy caused stickiness and clogging in the apparatus and was favorable in handling properties.

(Production Example 4)

To a twin-screw kneader whose rotational speed was modulated to 100 rpm, were added 130 parts of water and a solution of 20 parts of sodium hydroxide in 100 parts of water, and hardwood pulp (manufactured by Nippon Paper Industries Co., Ltd., LBKP) was added thereto in an amount of 100 parts in terms of dry mass in drying at 100°C for 60 minutes. The resultant was stirred and mixed at 30°C for 90 minutes, thereby preparing mercerized cellulose. After 100 parts of isopropanol (IPA) and 60 parts of sodium monochloroacetate were added with further stirring and the resultant was stirred for 30 minutes, the temperature was raised to 70°C, thereby allowing for a carboxymethylation reaction for 90 minutes. The concentration of IPA in the reaction medium in the carboxymethylation reaction was 30%. After completion of the reaction, the resultant was neutralized with acetic acid to a pH of about 7, and subjected to liquid removal, drying, and pulverization, thereby obtaining a pulverized product of a carboxymethylated pulp sodium salt having a degree of carboxymethyl substitution of 0.24 and a degree of crystallization of cellulose type I of 73%. The rate of effective utilization of the carboxymethylating agent was 29%. The methods for measuring the degree of carboxymethyl substitution and the degree of crystallization of cellulose type I, and the method for calculating the rate of effective utilization of the carboxymethylating agent are as described above.

(Production Example 5)

A pulverized product of a carboxymethylated pulp sodium salt was obtained in the same manner as in Production Example 4 except that the amount of IPA added was changed to thereby set the concentration of IPA in the reaction liquid in the carboxymethylation reaction to 50%. The degree of carboxymethyl substitution was 0.31, the degree of crystallization of cellulose type I was 66%, and the rate of effective utilization of the carboxymethylating agent was 37%.

(Production Example 6)

A pulverized product of a carboxymethylated pulp sodium salt was obtained in the same manner as in Production Example 4 except that the amount of IPA added was changed to thereby set the concentration of IPA in the reaction liquid in the carboxymethylation reaction to 65%. The degree of carboxymethyl substitution was 0.20, the degree of crystallization of cellulose type I was 74%, and the rate of effective utilization of the carboxymethylating agent was 25%.

(Production Examples 7 to 10)

The pulverized products of Production Examples 7 to 10 corresponded to the pulverized products of fibrillated carboxymethylated pulp of Examples 1 to 4, respectively.

(Production Examples 11 to 14)

The pulverized products of Production Examples 11 to 14 were obtained by drying the pulverized products of fibrillated carboxymethylated pulp of Production Examples 7 to 10, respectively, by a freeze-drying method.

(Comparative Production Example 1)

A pulverized product of a carboxymethylated pulp sodium salt was obtained in the same manner as in Production Example 4 except that a solution of 45 parts of sodium hydroxide in 100 parts of water was used in the mercerization reaction, instead of the solution of 20 parts of sodium hydroxide in 100 parts of water, the solvent in the carboxymethylation reaction was 100% water, and 150 parts of sodium monochloroacetate was used as the carboxymethylating agent, instead of 60 parts of sodium monochloroacetate. The degree of carboxymethyl substitution was 0.28, the degree of crystallization of cellulose type I was 45%, and the rate of effective utilization of the carboxymethylating agent was 13%.

(Comparative Production Example 2)

A pulverized product of a carboxymethylated pulp sodium salt was obtained in the same manner as in Production Example 4 except that dissolving pulp (manufactured by Nippon Paper Industries Co., Ltd., NDPS) was used instead of the hardwood pulp, a solution of 500 parts of IPA and 48 parts of sodium hydroxide in 100 parts of water was used in the mercerization reaction, and a solution of 37 parts of monochloroacetic acid in 45 parts of 90%IPA was used in the carboxymethylation reaction. The degree of carboxymethyl substitution was 0.50, the degree of crystallization of cellulose type I was 43%, and the rate of effective utilization of the carboxymethylating agent was 78.8%.

Each dried pulverized product of carboxymethylated pulp of Production Examples 4 to 6 was subjected to measurements of the proportion of the filtration residue, the Schopper-Riegler freeness, the Canadian standard freeness, the amount of drainage, the viscosity (25°C, 30 rpm) of the water dispersion, and the degree of anionization, according to the methods described in the section "Carboxymethylated pulp" as the measurement methods of the carboxymethylated pulp. The results are shown in Table 2.

[Table 2]

|  |  | Production Example 4 | Production Example 5 | Production Example 6 |
|---|---|---|---|---|
| Solvent in mercerization | Water | 100% | 100% | 100% |
|  | Organic solvent | - | - | - |
| Solvent in carboxymethylation | Water | 70% | 50% | 30% |
|  | Organic solvent | IPA 30% | IPA 50% | IPA 70% |
| Rate of effective utilization of carboxymethylating agent |  | 29% | 37% | 25% |
| Degree of carboxymethyl substitution |  | 0.24 | 0.31 | 0.20 |
| Degree of crystallization of cellulose type I |  | 73% | 66% | 74% |
| Proportion of filtration residue |  | 7% | 2% | 3% |
| Schopper-Riegler freeness (°SR) |  | 66.7 | 71.3 | Not measured |
| Canadian standard freeness (ml) |  | 106 | 85 | Not measured |
| Amount of drainage (ml/10 seconds) |  | 369 | 302 | Not measured |
| Viscosity at 1% (25°C, 30 rpm) (mPa·s) |  | 5.6 | 5.6 | Not measured |
| Degree of anionization |  | 0.32 | 0.53 | Not measured |

(Examples 5 to 11, and Comparative Examples 2 and 3: breads)

Each dough of Examples and Comparative Examples was prepared by formulation as represented below. Thereafter, such each resulting dough was fermented and baked according to steps of a usual straight method, thereby obtaining each square bread. Such each resulting bread was sensorily evaluated by ten trained panelists, with respect to the water retention ability after baking. The results are shown in Table 3.

Formulation of dough for bread

| Wheat flour | 100.0 parts |
|---|---|
| Yeast | 2.0 parts |
| Yeast food | 0.05 parts |
| Sugar | 7.0 parts |
| Salt | 2.0 parts |
| Skimmed milk powder | 2.0 parts |
| Shortening | 4.0 parts |
| Each pulverized product of carboxymethylated pulp (solid content) | 0.5 parts |
| Water | 72.0 parts. |

Sensory evaluation with respect to water retention ability

The water retention ability (moist texture (mouthfeel)) of such each resulting bread was evaluated as either good or poor by ten trained panelists. The results are shown in Table 3. The symbols of "Good", "Fair", and "Poor" in Table 3 represent the following evaluation results:

Good: nine panelists or more out of ten panelists evaluated the water retention ability (moist texture) as good

Fair: six to eight panelists out of ten panelists evaluated the water retention ability as good

Poor: five panelists or less out of ten panelists evaluated the water retention ability as good.

Evaluation with respect to shape retention ability

The shape retention ability of such each resulting bread was determined by measuring the volume with a laser volumeter VM-2000V (Astaix Inc.) before and after heating with a toaster, and the rate of volume reduction was calculated from the resulting value, and rated according to the following criteria:

Good: the rate of volume reduction after heating with a toaster was 7% or less

Fair: the rate of volume reduction after heating with a toaster was more than 7% to 9% or less

Poor: the rate of volume reduction after heating with a toaster was more than 9%.

[Table 3]

|  | Pulverized product | Water retention ability (moist texture) | Shape retention ability |
|---|---|---|---|
| Example 5 | Production Example 4 | Good | Good |
| Example 6 | Production Example 5 | Good | Good |
| Example 7 | Production Example 6 | Good | Good |
| Example 8 | Production Example 7 | Good | Good |
| Example 9 | Production Example 8 | Good | Good |
| Example 10 | Production Example 9 | Good | Good |
| Example 11 | Production Example 10 | Good | Good |
| Com parative Example 2 | Comparative Production Example 1 | Fair | Poor |
| Com parative Example 3 | Comparative Production Example 2 | Poor | Poor |

As clear from the results in Table 3, it has been found that any additive including the pulverized product of carboxymethylated pulp of the present invention imparts a moist texture to a bread and is suitable as an agent imparting water retention ability, for use in food products.

(Examples 12 to 18, and Comparative Examples 4 and 5: gummi candy)

Each gummi candy stock solution of Examples and Comparative Examples was prepared by formulation as represented below. A mold made of PP (length × width × height = 20 mm × 20 mm × 15 mm) was filled with the resulting gummi candy stock solution so that the height was 10 mm. A mesh having a diameter of 20 cm and a height of 6 cm was placed in a pan having a diameter of 24 cm and a height of 14 cm dedicated for an IH heater so that the bottom of the mesh faced upward, and 1 L of water was placed therein. The pan was heated with the IH heater, and the heater was set so as to keep its temperature when water was boiled and steam started to be generated. The internal temperature of the pan was here 100°C. The mold made of PP, filled with the gummi candy stock solution, was placed on the bottom of the mesh, a lid was closed so that a wet cloth was sandwiched between the pan and the lid, and the resultant was steam-heated for 30 minutes, thereby obtaining each gummi candy. Such each resulting gummi candy was evaluated with respect to textures and sticky feeling. The results are shown in Table 4.

Formulation of gummi candy stock solution

| Reduced starch syrup | 49.4 parts |
|---|---|
| Powder sugar | 42.8 parts |
| Each pulverized product of carboxymethylated pulp | 6.4 parts |
| Citric acid | 1.2 parts |
| Grape flavor | 0.2 parts |

Evaluation of textures

The mouthfeel textures (firm textures, juiciness) of such each resulting gummi candy were evaluated as either good or poor by ten trained panelists. The results are shown in Table 4. The symbols of "Good", "Fair", and "Poor" in Table 4 represent the following evaluation results:

Good: nine panelists or more out of ten panelists evaluated the textures as good Fair: six to eight panelists out of ten panelists evaluated the textures as good

Poor: five panelists or less out of ten panelists evaluated the textures as good.

Evaluation of sticky feeling

The degree of stickiness was sensorily evaluated by touching such each resulting gummi candy. The criteria are as follows:

Good: not sticky at all

Fair: almost not sticky

Poor: strong sticky feeling.

[Table 4]

| | Pulverized product of CMC | Textures (firm textures, juiciness) | Sticky feeling |
|---|---|---|---|
| Example 12 | Production Example 4 | Good | Good |
| Example 13 | Production Example 5 | Good | Good |
| Example 14 | Production Example 6 | Good | Good |
| Example 15 | Production Example 11 | Good | Good |
| Example 16 | Production Example 12 | Good | Good |
| Example 17 | Production Example 13 | Good | Good |
| Example 18 | Production Example 14 | Good | Good |
| Com parative Example 4 | Comparative Production Example 1 | Fair | Good |
| Com parative Example 5 | Comparative Production Example 2 | Poor | Poor |

It has been found from the results in Table 4 that any additive including the pulverized product of carboxymethylated pulp of the present invention can impart firm resilience to a gummi candy and is suitable as an agent imparting shape retention ability, for use in food products. It has also been found that such each additive not only imparts a juicy texture, but also is hardly sticky, and is suitable as an agent imparting water retention ability, for use in food products.

(Examples 19 to 25, and Comparative Examples 6 and 7: probiotic drinks)

Each pulverized product of carboxymethylated pulp was added to granulated sugar and 70% isomerized liquid sugar, in a predetermined amount calculated so that the following formulation was achieved, and water was added thereto for complete dissolution. The dissolved liquid was sterilized at 80°C for 10 minutes and cooled to 20°C $\pm$ 1°C, thereafter a predetermined amount of fermented milk was added thereto, and the resultant was mixed and stirred. The resultant was allowed to pass through a homogenizer at 150 kg/cm$^2$ once. The mixed and stirred liquid homogenized was sterilized at 90°C and then cooled to 20°C, and 2.0 ml of 7% sodium benzoate was further added for corruption prevention, thereby obtaining each probiotic drink of Examples and Comparative Examples. Such each resulting drink was evaluated with respect to textures and dispersion stability. The results are shown in Table 5.

Formulation of probiotic drink

| | |
|---|---|
| Fermented milk (on anhydrous basis) | 3.0 parts |
| Granulated sugar | 1.5 parts |
| 70% Isomerized liquid sugar | 9.3 parts |
| Each pulverized product of carboxymethylated pulp (solid content) | 0.5 parts |
| Water | 85.7 parts |

Evaluation of textures

The mouthfeel textures (smoothness, less gooeyness and clump) of such each resulting probiotic drink were evaluated as either good or poor by ten trained panelists. The results are shown in Table 5. The symbols of "Good", "Fair", and "Poor" in Table 5 represent the following evaluation results:

Good: nine panelists or more out of ten panelists evaluated the textures as good Fair: six to eight panelists out of ten panelists evaluated the textures as good

Poor: five panelists or less out of ten panelists evaluated the textures as good.

Evaluation of dispersion stability

Such each resulting probiotic drink was placed in a 100-ml graduated cylinder and left to still stand for 2 weeks, and the amount of precipitation of milk protein in such a cylindrical tube after 2 weeks was read. It was indicated that, as the value was smaller, the stability of such each probiotic drink was more excellent. The criteria are as follows:

Good: the amount of precipitation was less than 5.0 ml

Fair: the amount of precipitation was 5.0 ml or more and less than 8.0 ml

Poor: the amount of precipitation was 8.0 ml or more.

[Table 5]

| | | Pulverized product of CMC | Textures (smoothness, less gooeyness and clump) | Dispersion stability |
|---|---|---|---|---|
| | Example 19 | Production Example 4 | Good | Good |
| | Example 20 | Production Example 5 | Good | Good |
| | Example 21 | Production Example 6 | Good | Good |
| | Example 22 | Production Example 7 | Good | Good |

(continued)

|  | Pulverized product of CMC | Textures (smoothness, less gooeyness and clump) | Dispersion stability |
|---|---|---|---|
| Example 23 | Production Example 8 | Good | Good |
| Example 24 | Production Example 9 | Good | Good |
| Example 25 | Production Example 10 | Good | Good |
| Com parative Example 6 | Comparative Production Example 1 | Fair | Poor |
| Com parative Example 7 | Comparative Production Example 2 | Poor | Fair |

It has been found from the results in Table 5 that any additive containing the carboxymethylated pulp in the present invention is excellent in dispersion stabilization of a probiotic drink, and is suitable as a dispersion stabilizer for use in food products. It has also been found that such any additive has smoothness to the throat and is less in gooeyness and clump, and can be used as a viscosity modifier for use in food products, which is less in gooeyness.

(Examples 26 to 32, and Comparative Examples 8 and 9: chocolate beverage)

A cocoa powder, sugar, a skimmed milk powder, and each pulverized product of carboxymethylated pulp were added in predetermined amounts calculated so that the following formulation was achieved, water was added thereto, and the resultant was heated up to 80°C with stirring by a homomixer and thus preliminarily emulsified, and homogenized by a homogenizer at a pressure of 300 kgf/cm$^2$. Thereafter, a can was filled therewith, and the resultant was sterilized at 121°C for 30 minutes, thereby obtaining each chocolate beverage of Examples and Comparative Examples. Such each resulting chocolate beverage was evaluated with respect to textures and dispersion stability. The results are shown in Table 6.

Formulation of chocolate beverage

| Cocoa powder | 4.0 parts |
| Sugar | 10.0 parts |
| Skimmed milk powder | 4.0 parts |
| Each pulverized product of carboxymethylated pulp (solid content) | 2.0 parts |
| Water | 80.0 parts |

Evaluation of textures

The mouthfeel textures (smoothness, low roughness) of such each resulting chocolate beverage were evaluated as either good or poor by ten trained panelists. The results are shown in Table 6. The symbols of "Good", "Fair", and "Poor" in Table 6 represent the following evaluation results:

Good: nine panelists or more out of ten panelists evaluated the textures as good
Fair: six to eight panelists out of ten panelists evaluated the textures as good
Poor: five panelists or less out of ten panelists evaluated the textures as good.

Evaluation of dispersion stability

Such each resulting chocolate beverage was placed in a 100-ml graduated cylinder and left to still stand for 2 weeks, and the amount of precipitation of milk protein in such a cylindrical tube after 2 weeks was read. It was indicated that, as the value was smaller, the stability of such each chocolate beverage was more excellent. The criteria are as follows:

Good: the amount of precipitation was less than 5.0 ml

Fair: the amount of precipitation was 5.0 ml or more and less than 8.0 ml

Poor: the amount of precipitation was 8.0 ml or more

[Table 6]

| | Pulverized product of CMC | Textures (smoothness, low roughness) | Dispersion stability |
|---|---|---|---|
| Example 26 | Production Example 4 | Good | Good |
| Example 27 | Production Example 5 | Good | Good |
| Example 28 | Production Example 6 | Good | Good |
| Example 29 | Production Example 7 | Good | Good |
| Example 30 | Production Example 8 | Good | Good |
| Example 31 | Production Example 9 | Good | Good |
| Example 32 | Production Example 10 | Good | Good |
| Com parative Example 8 | Comparative Production Example 1 | Fair | Poor |
| Com parative Example 9 | Comparative Production Example 2 | Poor | Fair |

It has been found from the results in Table 6 that any additive including the pulverized product of carboxymethylated pulp of the present invention is excellent in dispersion stabilization of a chocolate beverage, can impart a smooth texture low in roughness, to a beverage, and is suitable as a dispersion stabilizer for use in food products.

(Examples 33 to 39, and Comparative Examples 10 and 11: dispersion stability of cocoa powder)

The dispersion stability was visually observed in a case where 5 parts of each pulverized product of carboxymethylated pulp was added to 100 parts of an aqueous 20% solution of commercially available powder cocoa (manufactured by Morinaga & Co., Ltd.). Further, after still standing for 24 hours, re-stirring was made and the re-dispersibility was visually observed. Both dispersion stability and re-dispersibility were evaluated as follows: a case where no precipitate was observed on the bottom of a storage container was rated as "Good", a case where a precipitate was slightly observed on a portion of the bottom of a storage container was rated as "Fair", and a case where a precipitate was observed entirely on the bottom of a storage container was rated as "Poor". The results are shown in Table 7.

[Table 7]

| | Pulverized product of CMC | Dispersion stability | Re-dispersibility |
|---|---|---|---|
| Example 33 | Production Example 4 | Good | Good |
| Example 34 | Production Example 5 | Good | Good |
| Example 35 | Production Example 6 | Good | Good |
| Example 36 | Production Example 11 | Good | Good |
| Example 37 | Production Example 12 | Good | Good |
| Example 38 | Production Example 13 | Good | Good |
| Example 39 | Production Example 14 | Good | Good |
| Com parative Example 10 | Comparative Production Example 1 | Poor | Poor |
| Com parative Example 11 | Comparative Production Example 2 | Fair | Fair |

It has been found from the results in Table 7 that any additive containing the pulverized product of carboxymethylated pulp of the present invention is excellent in dispersion stabilization and re-dispersibility of a cocoa beverage, and is suitable as a dispersion stabilizer for use in food products.

(Examples 40 to 46, and Comparative Examples 12 and 13: pudding)

A powdery mixture of raw materials other than a pudding flavor, formulated as represented below, was added with water and fresh cream being stirred, the resultant was stirred and dissolved at 80°C for 10 minutes, thereafter the pudding flavor was added thereto, a container was filled therewith, and the resultant was cooled, thereby adjusting each pudding of Examples and Comparative Examples. Thereafter, the shape retention ability and texture of such each pudding taken out from the container were evaluated. The results are shown in Table 8.

Formulation of pudding

| Fresh cream | 5.0 parts |
| Sugar | 10.0 parts |
| Skimmed milk powder | 8.0 parts |
| Each pulverized product of carboxymethylated pulp (solid content) | 0.3 parts |
| Pudding flavor | 0.1 parts |
| Water | 77.0 parts |

Evaluation of texture

The mouthfeel texture (smoothness) of such each resulting pudding was evaluated as either good or poor by ten trained panelists. The results are shown in Table 8. The symbols of "Good", "Fair", and "Poor" in Table 8 represent the following evaluation results:

Good: nine panelists or more out of ten panelists evaluated the texture as good

Fair: six to eight panelists out of ten panelists evaluated the texture as good

Poor: five panelists or less out of ten panelists evaluated the texture as good.

Evaluation of shape retention ability

Such each resulting pudding was taken out from the container, and whether or not the shape collapsed was visually evaluated according to the following criteria.

Good: the same shape as that of the container was almost kept

Poor: the shape significantly collapsed under its own weight.

[Table 8]

|  | Pulverized product of CMC | Texture (smoothness) | Shape retention ability |
|---|---|---|---|
| Example 40 | Production Example 4 | Good | Good |
| Example 41 | Production Example 5 | Good | Good |
| Example 42 | Production Example 6 | Good | Good |
| Example 43 | Production Example 7 | Good | Good |
| Example 44 | Production Example 8 | Good | Good |
| Example 45 | Production Example 9 | Good | Good |
| Example 46 | Production Example 10 | Good | Good |
| Com parative Example 12 | Com parative Production Example 1 | Fair | Poor |

(continued)

| | Pulverized product of CMC | Texture (smoothness) | Shape retention ability |
|---|---|---|---|
| Com parative Example 13 | Com parative Production Example 2 | Poor | Poor |

It has been found from the results in Table 8 that any additive containing the pulverized product of carboxymethylated pulp of the present invention not only imparts sufficient shape retention ability to a pudding, but also is kept in smooth texture, and is suitable as an emulsion stabilizer and an agent imparting shape retention ability, for use in food products.

(Examples 47 to 53, and Comparative Examples 14 and 15: jelly)

A powdery mixture of sugar, each pulverized product of carboxymethylated pulp, trisodium citrate, and calcium lactate formulated as represented below was added with water being stirred, the resultant was heated, stirred, and dissolved at 80°C for 10 minutes, thereafter citric acid (anhydrous) was added thereto, the resultant was stirred and mixed, the total amount of the resulting mixture was corrected by water, a container was filled with the mixture, and the mixture was sterilized at 85°C for 30 minutes and solidified by water cooling, thereby producing each jelly of Examples and Comparative Examples. Thereafter, the shape retention ability and textures of such each jelly taken out from the container were evaluated. The results are shown in Table 9.

Formulation of jelly

| | |
|---|---|
| Sugar | 15.0 parts |
| Citric acid | 0.2 parts |
| Each pulverized product of carboxymethylated pulp (solid content) | 0.3 parts |
| Trisodium citrate | 0.2 parts |
| Calcium lactate | 0.2 parts |
| Water | 84.0 parts |

Evaluation of textures

The mouthfeel textures (proper resilience, juiciness) of such each resulting jelly were evaluated as either good or poor by ten trained panelists. The results are shown in Table 9. The symbols of "Good", "Fair", and "Poor" in Table 9 represent the following evaluation results:

Good: nine panelists or more out of ten panelists evaluated the textures as good
Fair: six to eight panelists out of ten panelists evaluated the textures as good
Poor: five panelists or less out of ten panelists evaluated the textures as good.

Evaluation of shape retention ability

Such each resulting jelly was taken out from the container, and whether or not the shape collapsed was visually evaluated according to the following criteria.

Good: the same shape as that of the container was almost maintained

Poor: the shape significantly collapsed under its own weight.

[Table 9]

| | Pulverized product of CMC | Textures (proper resilience, juiciness) | Shape retention ability |
|---|---|---|---|
| Example 47 | Production Example 4 | Good | Good |

(continued)

| | Pulverized product of CMC | Textures (proper resilience, juiciness) | Shape retention ability |
|---|---|---|---|
| Example 48 | Production Example 5 | Good | Good |
| Example 49 | Production Example 6 | Good | Good |
| Example 50 | Production Example 7 | Good | Good |
| Example 51 | Production Example 8 | Good | Good |
| Example 52 | Production Example 9 | Good | Good |
| Example 53 | Production Example 10 | Good | Good |
| Com parative Example 14 | Com parative Production Example 1 | Poor | Poor |
| Com parative Example 15 | Com parative Production Example 2 | Poor | Poor |

It has been found from the results in Table 9 that any additive containing the pulverized product of carboxymethylated pulp of the present invention can impart to each jelly, not only sufficient shape retention ability and proper resilience, but also a juicy texture, and is suitable as an agent imparting shape retention ability and an agent imparting water retention ability, for use in food products.

(Examples 54 to 60, and Comparative Examples 16 and 17: hamburger steak)

After comminuted meat, an onion, breadcrumbs, an egg, black pepper, common salt, and water formulated as represented below were mixed by an SK mixer for 3 minutes, each pulverized product of carboxymethylated pulp was added thereto and well mixed, and 100 g of each oval shaped article was formed. Such each resulting hamburger steak was cooked in a pan so that both surfaces of the hamburger steak were on high heat for 2 minutes and thereafter were on low heat with a lid for 12 minutes in total, thereby adjusting each hamburger steak of Examples and Comparative Examples. The shape retention ability and textures of such each resulting hamburger steak were evaluated. The results are shown in Table 10.

Formulation of hamburger steak

| Comminuted meat | 57.9 parts |
|---|---|
| Onion | 21.1 parts |
| Breadcrumbs | 10.5 parts |
| Egg | 6.3 parts |
| Black pepper | 0.1 parts |
| Common salt | 0.8 parts |
| Each pulverized product of carboxymethylated pulp | 0.5 parts |
| Water | 3.2 parts |

Evaluation of textures

The mouthfeel textures (proper firmness, smoothness) of such each resulting hamburger steak were evaluated as either good or poor by ten trained panelists. The results are shown in Table 10. The symbols of "Good", "Fair", and "Poor" in Table 10 represent the following evaluation results:

Good: nine panelists or more out of ten panelists evaluated the textures as good

Fair: six to eight panelists out of ten panelists evaluated the textures as good

Poor: five panelists or less out of ten panelists evaluated the textures as good.

Evaluation of shape retention ability

The shape retention ability of such each hamburger steak during cooking was evaluated according to the following criteria.

Good: the shape hardly collapsed

Poor: the shape easily collapsed.

[Table 10]

|  | Pulverized product of CMC | Textures (proper firmness, smoothness) | Shape retention ability |
|---|---|---|---|
| Example 54 | Production Example 4 | Good | Good |
| Example 55 | Production Example 5 | Good | Good |
| Example 56 | Production Example 6 | Good | Good |
| Example 57 | Production Example 11 | Good | Good |
| Example 58 | Production Example 12 | Good | Good |
| Example 59 | Production Example 13 | Good | Good |
| Example 60 | Production Example 14 | Good | Good |
| Com parative Example 16 | Comparative Production Example 1 | Fair | Poor |
| Com parative Example 17 | Comparative Production Example 2 | Poor | Poor |

It has been found from the results in Table 10 that any additive containing the pulverized product of carboxymethylated pulp of the present invention can impart sufficient shape retention ability and also favorable textures to a hamburger steak, and is suitable as, for example, an agent imparting shape retention ability, for use in food products.

(Examples 61 to 67, and Comparative Examples 18 and 19: textures of pancake and bread)

Milk and an egg were added to a commercially available pancake mix (Pancake Mix manufactured by Nippon Flour Mills Co., Ltd.), 1% by mass of each pulverized product of carboxymethylated pulp was added thereto, the resultant was baked on a hot plate (160°C, 5 minutes) after 5 minutes, and the moist feeling of each pancake was evaluated by ten panelists immediately after such cooking and after 20 hours of such cooking.

Common salt, sugar, milk, an egg, butter, and dry yeast were added to commercially available bread flour (brand: Nisshin Camellia), 1% by mass of each pulverized product of carboxymethylated pulp was added thereto, each bread roll was produced according to an ordinary method, and the moist feeling of such each bread roll was evaluated by ten panelists immediately after such production and after 20 hours of such production.

The results are shown in Table 11. The symbols of "Good", "Fair", and "Poor" in Table 11 represent the following evaluation results:

Good: nine panelists or more out of ten panelists evaluated the texture (moist texture, mouthfeel) as good

Fair: six to eight panelists out of ten panelists evaluated the texture as good

Poor: five panelists or less out of ten panelists evaluated the texture as good.

[Table 11]

| | Pulverized product of CMC | Pancake | | Bread roll | |
|---|---|---|---|---|---|
| | | Immediate aftermath | After 20 hours | Immediate aftermath | After 20 hours |
| Example 61 | Production Example 4 | Good | Good | Good | Good |
| Example 62 | Production Example 5 | Good | Good | Good | Good |
| Example 63 | Production Example 6 | Good | Good | Good | Good |
| Example 64 | Production Example 11 | Good | Good | Good | Good |
| Example 65 | Production Example 12 | Good | Good | Good | Good |
| Example 66 | Production Example 13 | Good | Good | Good | Good |
| Example 67 | Production Example 14 | Good | Good | Good | Good |
| Com parative Example 18 | Comparative Production Example 1 | Fair | Poor | Fair | Poor |
| Com parative Example 19 | Comparative Production Example 2 | Fair | Poor | Poor | Poor |

It has been found from the results in Table 11 that any additive including the pulverized product of carboxymethylated pulp of the present invention can impart a moist texture to a pancake and/or a bread roll over a long time, and is suitable as, for example, an agent imparting water retention ability, for use in food products.

(Examples 68 to 74, and Comparative Examples 20 and 21: milky lotion (cosmetic product))

Each milky lotion (cosmetic product) of Examples and Comparative Examples was produced by formulation as represented below. Such each resulting milky lotion was evaluated with respect to emulsion stability, non-rough feeling, non-sticky feeling, spreadability, moisture retention ability, and attachment ability. The results are shown in Table 12.

Formulation of milky lotion

| | |
|---|---|
| Stearic acid | 4.0 parts |
| Squalane | 5.0 parts |
| Glycerin | 5.0 parts |
| Propylene glycol | 5.0 parts |
| Sucrose fatty acid ester | 2.0 parts |
| Each pulverized product of carboxymethylated pulp (solid content) | 3.0 parts |
| Water | 70.0 parts |

Evaluation of emulsion stability

Such each milky lotion was left to still stand at room temperature for 1 week, and a case where no precipitate was observed on the bottom of a storage container was rated as "Good" and a case where any precipitate was observed thereon was rated as "Poor".

Evaluation of non-rough feeling, non-sticky feeling, spreadability, moisture retention ability, and attachment ability

The non-rough feeling, non-sticky feeling, spreadability, moisture retention ability, and attachment ability of such each resulting milky lotion were evaluated as either good or poor by fifteen trained female panelists. The results are shown in Table 12. The symbols of "Good", "Fair", and "Poor" in Table 12 represent the following evaluation results:

Good: eleven panelists or more out of fifteen panelists evaluated such properties as good

Fair: six to ten panelists out of fifteen panelists evaluated such properties as good

Poor: five panelists or less out of fifteen panelists evaluated such properties as good.

[Table 12]

|  | Pulverized product of CMC | Non-rough feeling | Non-sticky feeling | Spreadability | Moisture retention ability | Attachment ability |
|---|---|---|---|---|---|---|
| Example 68 | Production Example 4 | Good | Good | Good | Good | Good |
| Example 69 | Production Example 5 | Good | Good | Good | Good | Good |
| Example 70 | Production Example 6 | Good | Good | Good | Good | Good |
| Example 71 | Production Example 7 | Good | Good | Good | Good | Good |
| Example 72 | Production Example 8 | Good | Good | Good | Good | Good |
| Example 73 | Production Example 9 | Good | Good | Good | Good | Good |
| Example 74 | Production Example 10 | Good | Good | Good | Good | Good |
| Comparative Example 20 | Comparative Production Example 1 | Fair | Fair | Poor | Poor | Poor |
| Comparative Example 21 | Comparative Production Example 2 | Poor | Poor | Poor | Fair | Fair |

It has been found from the results in Table 12 that any additive containing the pulverized product of carboxymethylated pulp of the present invention imparts emulsion stability, low-rough feeling and low-sticky feeling, and also spreadability, moisture retention ability, and favorable attachment ability to a milky lotion, and is suitable as an emulsion stabilizer, an agent imparting water retention ability, and a viscosity modifier, which are for use in cosmetic products.

(Examples 75 to 81, and Comparative Examples 22 to 24: feed pellet)

Each pulverized product of carboxymethylated pulp was prepared by adding water so that the moisture percentage was 30%, and thereafter treated in a die having a diameter of 4.8 mm and an effective thickness of 32 mm in a ring die-type small size pelletizer (manufactured by CPM), thereby producing a feed pellet. In Comparative Example 24, softwood pulp was dehydrated so that the moisture percentage was 30%, and thereafter treated in a die having a diameter of 4.8 mm and an effective thickness of 32 mm in a ring die-type small size pelletizer (manufactured by CPM), thereby producing a feed pellet.

Rate of cellulase saccharification

The rate of cellulase saccharification of such each resulting feed pellet was measured according to the following method:
A molded feed product (absolute dry mass: 500 mg) was accurately weighed and taken in a sample bottle made of resin (having a volume of 60 ml). Cellulase (trade name:
cellulase Onozuka p1500, manufactured by Yakult Pharmaceutical Industry Co., Ltd.) was added to a 0.1 M acetate buffer having a pH of 4.0 at a filter paper-disintegrating force of 1350 U/(absolute dry mass g of molded feed product) to provide a suspension, and 49.5 ml of the suspension was added to the container, and shaken with BioShaker BR-23FP manufactured by TAITEC CORPORATION, at 40°C and 180 rpm for 24 hours, thereby performing a saccharification treatment.
The sample was collected at a time point after 24 hours, and the proportion of any saccharificated molded feed product (rate of cellulase saccharification) was measured. Specifically, the sample was filtered on filter paper whose constant mass was determined in advance, and was washed with water four times and then dried in a forced-air drier at 105°C for 2 hours, and the mass of the dry content in the residue was measured. The rate of cellulase saccharification was calculated according to the following expression. The rate of cellulase saccharification highly correlates with the digestive efficiency in a ruminant animal, and a too high rate of cellulase saccharification after 24 hours causes the effect of promoting rumination to be lower, resulting in the risk of occurrence of ruminal acidosis. A rate of cellulase saccharification of 70% or more was rated as "Poor" (fail), and a rate of less than 70% was rated as "Good" (pass).

$$\text{Rate of cellulase saccharification (\%)} = [(\text{Mass of molded feed product before treatment with}$$

$$\text{cellulase - Mass of molded feed product (residue) after treatment with cellulase)/Mass of}$$

$$\text{molded feed product before treatment with cellulase}] \times 100$$

[Table 13]

|  | Raw material used | Rate of cellulase saccharification |
|---|---|---|
| Example 75 | Production Example 4 | Good |
| Example 76 | Production Example 5 | Good |
| Example 77 | Production Example 6 | Good |
| Example 78 | Production Example 11 | Good |
| Example 79 | Production Example 12 | Good |
| Example 80 | Production Example 13 | Good |
| Example 81 | Production Example 14 | Good |
| Comparative Example 22 | Comparative Production Example 1 | Poor |
| Comparative Example 23 | Comparative Production Example 2 | Poor |
| Comparative Example 24 | NBKP | Poor |

As shown in Table 13, it has been found that any feed containing the pulverized product of carboxymethylated pulp of the present invention is low in rate of cellulase saccharification after treatment for 24 hours and takes a longer time for saccharification, as compared with feeds of Comparative Examples. In other words, any feed pellet using the pulverized product of carboxymethylated pulp of the present invention can maintain a pellet shape and remain in the rumen of a ruminant animal for a longer time, and thus is considered to contribute to induction of rumination.

(Examples 82 to 88 and Comparative Examples 25 to 27: additive for rubber)

A water dispersion of each pulverized product of carboxymethylated pulp (solid content concentration: 1.0% by mass) in an amount corresponding to an absolute dry amount of 5% by mass was mixed with rubber latex (trade name: HA latex, manufactured by Regitex Co., Ltd., solid content concentration: 65% by mass) in an absolute dry solid content of 100 g, and the resultant was stirred with a TK homomixer (8000 rpm) for 60 minutes, thereby obtaining each mixture. The total solid content concentration of such each mixture was as very high as 68.25% by mass. Such each mixture was dried in a heating oven at 70°C for 10 hours, thereby obtaining each master batch.

Zinc oxide and stearic acid were mixed with such each master batch obtained according to the above method, in respective amounts of 6% by mass and 0.5% by mass relative to a rubber component in such each master batch, and the resultant was kneaded with an open roll (manufactured by Kansai Roll Co., Ltd.) at 30°C for 10 minutes, thereby obtaining each kneaded product. Sulfur and a vulcanization accelerator (BBS, N-t-butyl-2-benzothiazolesulfenamide) were added to such each kneaded product, in respective amounts of 3.5% by mass and 0.7% by mass relative to a rubber component in such each kneaded product, and the resultant was kneaded with an open roll (manufactured by Kansai Roll Co., Ltd.) at 30°C for 10 minutes, thereby obtaining each sheet of an unvulcanized rubber composition. Such each resulting sheet of an unvulcanized rubber composition was sandwiched in a mold, and press-vulcanized at 150°C for 10 minutes, thereby obtaining each vulcanized rubber sheet having a thickness of 2 mm. Such each resulting vulcanized rubber sheet was cut to a test piece having a predetermined shape, and the stress at a strain of 100% and the stress at a strain of 300%, and the stress at break, each exhibiting tensile strength, were measured according to JIS K6251 "Rubber, vulcanized or thermoplastic- Determination of tensile stress-strain properties".

In Comparative Example 27, the same as described above was also produced except that no pulverized product of carboxymethylated pulp was mixed in production of a master batch.

The results are shown in Table 14. The symbols of "Good" and "Poor" in Table 14 represent the following evaluation results.

Stress at strain of 100%

Good: 1.3 MPa or more

Poor: less than 1.3 MPa

Stress at strain of 300%

Good: 3.5 MPa or more

Poor: less than 3.5 MPa

Stress at break

Good: 23 MPa or more

Poor: less than 23 MPa

[Table 14]

|  |  | Pulverized product of CMC added | Stress at strain of 100% | Stress at strain of 300% | Stress at break |
|---|---|---|---|---|---|
|  | Example 82 | Production Example 4 | Good | Good | Good |
|  | Example 83 | Production Example 5 | Good | Good | Good |
|  | Example 84 | Production Example 6 | Good | Good | Good |
|  | Example 85 | Production Example 7 | Good | Good | Good |
|  | Example 86 | Production Example 8 | Good | Good | Good |
|  | Example 87 | Production Example 9 | Good | Good | Good |
|  | Example 88 | Production Example 10 | Good | Good | Good |
|  | Com parative Example 25 | Comparative Production Example 1 | Poor | Poor | Poor |
|  | Com parative Example 26 | Comparative Production Example 2 | Poor | Poor | Poor |
|  | Com parative Example 27 | None | Poor | Poor | Poor |

As shown in Table 14, it can be seen that any rubber to which the pulverized product of carboxymethylated pulp of the present invention is added is enhanced in strength as compared with each rubber of Comparative Examples.

**Claims**

1.   A pulverized product of carboxymethylated pulp having a degree of carboxymethyl substitution of 0.50 or less and having a degree of crystallization of cellulose type I of 50% or more.

2.   The pulverized product of carboxymethylated pulp according to claim 1, wherein pulverization is wet pulverization.

3.   The pulverized product of carboxymethylated pulp according to claim 1 or 2, fibrillated by pulverization.

4. The pulverized product of carboxymethylated pulp according to any one of claims 1 to 3, having an average fiber diameter of more than 500 nm.

5. The pulverized product of carboxymethylated pulp according to any one of claims 1 to 4, having a degree of anionization of 0.10 meq/g or more and 2.00 meq/g or less.

6. The pulverized product of carboxymethylated pulp according to any one of claims 1 to 5, having a degree of anionization of 1.00 meq/g or less.

7. The pulverized product of carboxymethylated pulp according to any one of claims 1 to 6, having a viscosity (25°C, 60 rpm) of 2500 mPa•s or less when in the form of a water dispersion having a solid content of 1% by mass.

8. The pulverized product of carboxymethylated pulp according to any one of claims 1 to 7, having a water retention capacity of 15 or more, as determined by preparing 40 mL of a slurry having a solid content of 0.3% by mass with water, then centrifuging the slurry by a centrifuge at 30°C and 25000 G for 30 minutes to thereby separate an aqueous phase and a sediment, and performing calculation according to the following expression:

Water retention capacity = (B + C - 0.003 $\times$ A)/(0.003 $\times$ A - C)
wherein A represents a mass of the slurry subjected to centrifugation, B represents a mass of the sediment separated, and C represents a mass of a solid content in the aqueous phase separated.

9. The pulverized product of carboxymethylated pulp according to any one of claims 1 to 8, having a structure obtained by linking carboxymethyl groups to at least a part of hydroxyl groups in glucose residues in cellulose to thereby form ether linkages.

10. An additive comprising the pulverized product of carboxymethylated pulp according to any one of claims 1 to 9.

11. The additive according to claim 10, as an additive for a food product, an additive for a pharmaceutical product, an additive for a cosmetic product, an additive for a feed, an additive for papermaking, an additive for a paint, an agent imparting water retention ability, an agent imparting shape retention ability, a viscosity modifier, an emulsion stabilizer, or a dispersion stabilizer.

12. A method for producing a pulverized product of carboxymethylated pulp having a degree of carboxymethyl substitution of 0.50 or less and having a degree of crystallization of cellulose type I of 50% or more, the method comprising a step of obtaining a pulverized product of carboxymethylated pulp by a mechanical treatment of carboxymethylated pulp.

13. The production method according to claim 12, wherein
the step of obtaining a pulverized product of carboxymethylated pulp by a mechanical treatment comprises fibrillating carboxymethylated pulp by a mechanical treatment, and
the pulverized product of carboxymethylated pulp comprises carboxymethylated pulp fibrillated.

14. The production method according to claim 12 or 13, further comprising a step of subjecting pulp to a mercerization reaction in a solvent containing mainly water and thereafter a carboxymethylation reaction in a mixed solvent of water and an organic solvent to thereby produce carboxymethylated pulp, before the step of obtaining a pulverized product of carboxymethylated pulp by a mechanical treatment.

15. The production method according to claim 14, wherein the solvent containing mainly water is a solvent comprising more than 50% by mass of water.

16. The production method according to claim 14 or 15, wherein a proportion of the organic solvent in the mixed solvent is 50 to 99% by mass based on the total of water and the organic solvent.

## INTERNATIONAL SEARCH REPORT

| International application No. |
| --- |
| PCT/JP2019/019670 |

**A. CLASSIFICATION OF SUBJECT MATTER**
Int. Cl. A23L29/00(2016.01)i, A23K20/163(2016.01)i, A61K8/73(2006.01)i, A61K47/38(2006.01)i, A61Q19/02(2006.01)i, C08B11/12(2006.01)i

According to International Patent Classification (IPC) or to both national classification and IPC

**B. FIELDS SEARCHED**

Minimum documentation searched (classification system followed by classification symbols)
Int. Cl. A23L29/00, A23K20/163, A61K8/73, A61K47/38, A61Q19/02, C08B11/12

Documentation searched other than minimum documentation to the extent that such documents are included in the fields searched
Published examined utility model applications of Japan      1922-1996
Published unexamined utility model applications of Japan    1971-2019
Registered utility model specifications of Japan            1996-2019
Published registered utility model applications of Japan    1994-2019

Electronic data base consulted during the international search (name of data base and, where practicable, search terms used)
JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| X | JP 10-251119 A (NIPPON PAPER INDUSTRIES CO., LTD.) 22 September 1998, claims, paragraph [0022], examples (Family: none) | 1-13 |
| X | JP 10-251301 A (NIPPON PAPER INDUSTRIES CO., LTD.) 22 September 1998, claims, examples, paragraph [0059] (Family: none) | 1-13 |
| X | JP 10-251446 A (NIPPON PAPER INDUSTRIES CO., LTD.) 22 September 1998, claims, example 1, paragraph [0059] (Family: none) | 1, 4-11 |
| X | JP 11-140793 A (NIPPON PAPER INDUSTRIES CO., LTD.) 25 May 1999, claims, example 1 (Family: none) | 1, 5-11 |

☒ Further documents are listed in the continuation of Box C.    ☐ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 21.06.2019 | 02.07.2019 |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

## INTERNATIONAL SEARCH REPORT

| International application No. |
|---|
| PCT/JP2019/019670 |

C (Continuation).   DOCUMENTS CONSIDERED TO BE RELEVANT

| Category* | Citation of document, with indication, where appropriate, of the relevant passages | Relevant to claim No. |
|---|---|---|
| X | WO 2014/087767 A1 (NIPPON PAPER INDUSTRIES CO., LTD.) 12 June 2014, claims, examples (Family: none) | 1-13 |
| X | JP 2015-149929 A (NIPPON PAPER INDUSTRIES CO., LTD.) 24 August 2015, examples 1, 2, paragraph [0019] (Family: none) | 1-13 |
| X | JP 2015-149930 A (NIPPON PAPER INDUSTRIES CO., LTD.) 24 August 2015, examples 1, 2, paragraph [0020] (Family: none) | 1-13 |
| P, X | JP 6337225 B1 (NIPPON PAPER INDUSTRIES CO., LTD.) 06 June 2018, examples, comparative examples (Family: none) | 1-3, 5-16 |
| P, X | JP 6351821 B1 (NIPPON PAPER INDUSTRIES CO., LTD.) 04 July 2018, examples, comparative examples (Family: none) | 1-3, 5-16 |
| P, X | JP 2018-164443 A (NIPPON PAPER INDUSTRIES CO., LTD.) 25 October 2018, examples (Family: none) | 1-13 |
| P, X | JP 6417490 B1 (NIPPON PAPER INDUSTRIES CO., LTD.) 07 November 2018, example 1, comparative example 2 (Family: none) | 1-3, 5-16 |
| P, X | JP 6442106 B1 (NIPPON PAPER INDUSTRIES CO., LTD.) 19 December 2018, claims, paragraph [0016], example 2 (Family: none) | 1-16 |
| P, X | JP 6505900 B1 (NIPPON PAPER INDUSTRIES CO., LTD.) 24 April 2019, entire text (Family: none) | 1-16 |
| P, X | JP 6505901 B1 (NIPPON PAPER INDUSTRIES CO., LTD.) 24 April 2019, entire text (Family: none) | 1-16 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017149901 A
- JP 2008222859 A
- JP 2007191558 A
- JP 2002194001 A
- WO 2014088072 A